# EUROPEAN PATENT APPLICATION

(11) **EP 4 729 614 A1**
(43) Date of publication of application: **22.04.2026**
(21) Application number: 24850979.6
(22) Date of filing: 06.08.2024
(51) Int. Cl.: C12N 15/10, B01D 15/10, B01D 15/16, B01D 15/38, C07H 21/02, C07H 1/06

(54) **SYSTEM AND METHOD FOR SEPARATING MRNA**

(30) Priority: 07.08.2023 WO PCT/CN2023/111429
(71) Applicant: Suzhou Abogen Biosciences Co., Ltd., Suzhou, Jiangsu 215123 (CN)
(72) Inventor: ZHANG, Xiaojing, Suzhou, Jiangsu 215123 (CN); WANG, Pei, Suzhou, Jiangsu 215123 (CN); GAO, Peng, Suzhou, Jiangsu 215123 (CN); YING, Bo, Suzhou, Jiangsu 215123 (CN)
(74) Representative: Prüfer & Partner mbB Patentanwälte · Rechtsanwälte
(86) International application number: PCT/CN2024/109945
(87) International publication number: WO 2025/031334

(57) **Abstract**

Provided is a method for separating mRNA molecules by means of a combination of magnetic particles and positive-pressure affinity chromatography. The combination of magnetic particles and positive-pressure affinity chromatography can be used to obtain high-yield and high-purity mRNA molecules from in vitro transcription. In particular, the method can be applied in an automated process, thereby obtaining high-quality mRNA molecules in a simplified high-throughput process.

## Description

### TECHNICAL FIELD

The present invention relates to the field of biological purification and, in particular, to a system and a method for separating mRNA.

### BACKGROUD

Messenger RNA (mRNA)-based therapeutics have shown great promise in preventing and treating various diseases. Compared to recombinant proteins expressed in mammalian cell lines, the production of mRNA is faster and more flexible as mRNA can be readily produced via in vitro transcription. However, the technical challenges faced by mRNA therapeutics are also clear. For example, the decreased efficacy of mRNA vaccines due to poor stability of RNA in solution and in vivo and limited expression of payload mRNA is a critical issue that requires careful optimization for preclinical and clinical applications (Sahin, U., Karikó, K. & Türeci, Ö. mRNA-based therapeutics-developing a new class of drugs. Nat. Rev. Drug Discov. 13, 759-780 (2014); Jackson, N. A. C., Kester, K. E., Casimiro, D., Gurunathan, S. & DeRosa, F. The promise of mRNA vaccines: a biotech and industrial perspective. npj Vaccines 5, 11 (2020); Weng, Y. et al. The challenge and prospect of mRNA therapeutics landscape. Biotechnol. Adv. 40, 107534 (2020); Crommelin, D. J. A., Anchordoquy, T. J., Volkin, D. B., Jiskoot, W. & Mastrobattista, E. Addressing the cold reality of mRNA vaccine stability. J. Pharm. Sci. 110, 997-1001 (2021)).

To solve the above problems, it is necessary to optimize the design of mRNA molecules, including 5' capping, promoter, 5' and 3' untranslated regions (UTR), Kozak sequence and signal domain, enhancer element, poly(A), intron and splice site, internal ribosome entry site, and CDS codon optimization, and the like (Guo, X., Wang, C., and Wang, T.Y. (2020). Chromatin-modifying elements for recombinant protein production in mammalian cell systems. Crit. Rev. Biotechnol.40, 1035-1043; Wang, T.Y., and Guo, X. (2020). Expression vector cassette engineering for recombinant therapeutic production in mammalian cell systems. Appl. Microbiol. Biotechnol. 104, 5673-5688; Mauro, V.P. (2018). Codon optimization in the production of recombinant biotherapeutics: potential risks and considerations. BioDrugs. 32, 6 9-81; Kawabe, Y., Inao, T., Komatsu, S., Huang, G., Ito, A., Omasa, T., and Kamihira, M. (2017). Improved recombinant antibody production by CHO cells using a production enhancer DNA element with repeated transgene integration at a predetermined chromosomal site. J. Biosci. Bioeng. 123, 390-397). Additionally, for the same vaccine project, it is necessary to screen effective sequences, which is a huge workload, cumbersome and changeable, and cannot be efficiently completed manually within a short timeframe.

In terms of process, modifications and reaction conditions (including NTP ratio, DNA template, reaction time, Mg ²⁺, and the like) of in vitro transcription are critical to the quality of the final mRNA stock solution, and usually optimized using the design of experiments (DOE) method. Due to the cumbersome changes in conditions, manual operations are prone to deviations. mRNA preparation and purification by an automated system can achieve high efficiency and controllably paralle results.

The mRNA product obtained by enzymatic transcription reaction will produce impurities such as truncated RNA, double-stranded (ds) RNA, and reactants, which need to be removed in the downstream purification process. A variety of techniques can be used to separate and purify mRNA, including, but not limited to, LiCl precipitation, chromatography, magnetic particles, and the like.

LiCl is commonly used for precipitating in vitro transcribed (IVT) RNA. LiCl precipitation employs high concentrations of lithium cations to selectively precipitate RNA. After mRNA precipitation, the supernatant containing salts, free NTPs, proteins, and DNAs was discarded. In this purification method, post-transcriptional DNase I treatment is optional to ensure complete removal of template DNA. LiCl precipitation provides a simple and rapid method for recovering RNA in an in vitro transcription reaction.

The mRNA purification process also often uses Oligo(dT) affinity chromatography to capture mRNA molecules and remove process-related impurities such as enzymes, NTPs and template DNAs. High-resolution hydrophobic or ion-exchange packing is used for fine purification to remove product-related impurities such as fragments or dsRNAs. Oligo(dT) affinity chromatography can remove most impurities such as NTPs, enzymes, and DNAs by binding to the Poly(A) tail for affinity purification, but cannot distinguish between dsRNAs and truncated RNAs. In addition, ion exchange chromatography (IEC), hydrophobic chromatography (HIC), ion-pair reversed-phase chromatography, and the like are also frequently used for mRNA purification.

Separation of biomolecules by using magnetic particles is known in the art. For example, Patent Publication No. WO2018122246 describes a method for separating biomolecules from a cell culture, comprising binding the biomolecules to magnetic beads, separating the magnetic beads comprising the bound biomolecules from the rest of the cell culture by using a magnetic separator, transporting the magnetic beads comprising the bound biomolecules as a slurry with added buffer to a separate elution unit, and eluting the biomolecules from the magnetic beads in the elution unit. The magnetic particles are used as an efficient way to purify target biomolecules directly from a crude feed unit stock, and this technique can replace clarification tools such as centrifugation, filtration, and capture chromatography. The magnetic particles are typically functionalized with ligands having affinity for target biomolecules. The magnetic particles are added and mixed with a crude feed for a certain period of time to specifically bind to all target biomolecules in the feed. After binding, the magnetic particles are captured by a magnet, while the cells and impurities are decanted away simultaneously. A wash buffer is added to the magnetic particles, the magnet is turned off, and the wash buffer and the magnetic particles are mixed. After mixing, the magnet is turned on to capture the magnetic particles and the wash buffer is decanted away. After several washes, an elution buffer is added to the magnetic particles in the same manner as the wash buffer, and the target biomolecules are released from the magnetic particles using several batch elution steps. All wash steps and elution steps are performed in batch mode, and since several batch elution steps need to be performed to obtain a high elution yield, the target will be more diluted with this technique compared to column chromatography. Furthermore, it is more difficult to optimize elution conditions using batch elution because the elution buffer requires titration of the wash buffer to achieve correct elution conditions to release the target biomolecules from the magnetic beads, and different or large volumes of elution buffer are required for each elution step. One way to overcome this is to transfer the washed magnetic particles into a chromatographic column for the elution step to reduce the volume of elution buffer and use an optimized elution buffer fractionation to collect the elution pool. However, this requires additional equipment and column packing, which is time-consuming.

At present, these technologies for separating and purifying mRNA have certain limitations and cannot fully meet the requirements for large-scale platform applications. They are also unable to provide mRNA-based therapeutics quickly in response to sudden epidemics. New tools and scalable workflows for mRNA vaccine development and production are critical to bringing this promising technology into clinical application. Accordingly, an improved method for separating mRNA molecules is required in the art, including purifying high-quality mRNA molecules from feed with reduced process time and in a more cost-effective manner.

### SUMMARY OF THE INVENTION

The present disclosure achieves the aforementioned objective of providing an improved method for separating and purifying mRNA molecules. The present disclosure relates to a method for separating mRNA molecules by means of a combination of magnetic particles and positive-pressure affinity chromatography. In particular, the method may be adapted in an automated process to increase throughput and shorten workflow to obtain high quality mRNA molecules for downstream applications.

After multiple rounds of testing, the present invention found that the combination of magnetic particles and positive-pressure affinity chromatography can be used to obtain high-yield and high-purity mRNA molecules from in vitro transcription. Without being bound to any theory, the magnetic particles and the positive-pressure affinity chromatography work synergistically with different principles of action, achieving a higher yield and purity than other methods or combination of methods for purifying mRNA with less time and material cost. Those skilled in the art will appreciate that the sequence and number of magnetic particles and positive-pressure affinity chromatography separation steps in the method of the present invention are not limited, whereby the methods of the present invention may comprise performing one or more magnetic particle separation steps and/or one or more positive-pressure affinity chromatography separation steps in any sequence or alternately.

In particular, in one embodiment, the method comprises one or more magnetic particle separation steps. In another embodiment, the method comprises one or more positive-pressure affinity chromatography separation steps. In one embodiment, the method comprises first performing one or more magnetic particle separation steps followed by one or more positive-pressure affinity chromatography separation steps. In another embodiment, the method comprises first performing one or more positive-pressure affinity chromatography separation steps followed by one or more magnetic particle separation steps. In one embodiment, the method comprises first performing one or more magnetic particle separation steps, then one or more positive-pressure affinity chromatography separation steps, followed by one or more magnetic particle separation steps. In another embodiment, the method comprises first performing one or more positive-pressure affinity chromatography separation steps, then performing one or more magnetic particle separation steps, followed by one or more positive-pressure affinity chromatography separation steps.

In a preferred embodiment, the method comprises first performing a magnetic particle separation step, then a positive-pressure affinity chromatography separation step, followed by optionally a magnetic particle separation step. In another preferred embodiment, the method comprises first performing a positive-pressure affinity chromatography separation step followed by a magnetic particle separation step.

More specifically, the present disclosure relates to a method for separating mRNA molecules, comprising the following steps of:
(a) providing magnetic particles comprising ligands capable of binding to the mRNA molecules;
(b) contacting a solution containing the mRNA molecules with the magnetic particles to obtain the magnetic particles comprising the bound mRNA molecules;
(c) separating the magnetic particles comprising the bound mRNA molecules using a magnetic field;
(d) eluting the mRNA molecules from the separated magnetic particles to obtain a primary purified solution containing the mRNA molecules; and
(e) loading the solution containing the mRNA molecules obtained in step (d) onto an affinity chromatography column comprising oligo (dT), and eluting the mRNA molecules in a positive-pressure manner to obtain a secondary purified solution containing the mRNA molecules.

In one embodiment, the method further comprises a step of performing in vitro transcription (IVT) on the DNA template prior to step (b) to generate a solution containing the mRNA molecules.

In one embodiment, the method further comprises a step of capping and/or tailing the mRNA molecules before step (b).

In one embodiment, the method further comprises a step of capping and/or tailing the mRNA molecules before step (e).

In one embodiment, the method further comprises a step of capping the mRNA molecules after step (e).

In one embodiment, the method further comprises a step of performing LiCl-based buffer exchange and concentration on a secondary purified solution containing the mRNA molecules after step (e).

In one embodiment, the method further comprises a step of performing magnetic particle-based buffer exchange and concentration on a secondary purified solution containing the mRNA molecules after step (e). In one embodiment, the magnetic particle method comprises repeating the steps (a)-(d).

In one embodiment, the method further comprises a step of sterilization and filtration of a secondary purified solution containing the mRNA molecules after step (e).

More specifically, the present disclosure relates to a method for separating mRNA molecules, comprising the following steps of:
(a) providing magnetic particles comprising ligands capable of binding to the mRNA molecules;
(b) performing in vitro transcription on a DNA template to generate a solution containing the mRNA molecules, wherein the mRNA molecule comprises a Poly(A) tail;
(c) contacting a solution containing the mRNA molecules with the magnetic particles to obtain magnetic particles comprising the bound mRNA molecules;
(d) separating the magnetic particles comprising the bound mRNA molecules using a magnetic field;
(e) eluting the mRNA molecules from the separated magnetic particles to obtain a primary purified solution containing the mRNA molecules;
(f) capping and incubating the mRNA molecules;
(g) loading the solution containing the mRNA molecules obtained in step (f) onto an affinity chromatography column comprising oligo (dT), and eluting the mRNA molecules in a positive-pressure manner to obtain a secondary purified solution containing the mRNA molecules; and
(h) performing buffer exchange and concentration and/or sterilization and filtration on the solution containing the mRNA molecules obtained in step (g).

In one embodiment, the buffer exchange and concentration in step (g) is performed by using a LiCl method or a magnetic particle method. In one embodiment, the magnetic particle method comprises repeating the steps (c)-(e).

More specifically, the present disclosure relates to a method for separating mRNA molecules, comprising the following steps of:
(a) providing a solution containing the mRNA molecules;
(b) loading the solution containing the mRNA molecules onto an affinity chromatography column comprising oligo (dT), eluting the mRNA molecules in a positive-pressure manner to obtain a primary purified solution containing the mRNA molecules;
(c) providing magnetic particles comprising ligands capable of binding to the mRNA molecules, and contacting the solution containing the mRNA molecules obtained in step (b) with the magnetic particles to obtain the magnetic particles comprising the bound mRNA molecules;
(d) separating the magnetic particles comprising the bound mRNA molecules using a magnetic field; and
(e) eluting the mRNA molecules from the separated magnetic particles to obtain a secondary purified solution containing the mRNA molecules.

In one embodiment, step (a) further comprises performing in vitro transcription on a DNA template to generate a solution containing the mRNA molecules.

In another embodiment, step (a) further comprises capping and/or tailing the mRNA molecules.

In another embodiment, the step after step (b) is to cap the mRNA molecules.

In another embodiment, the step after step (e) is to cap the mRNA molecules.

In one embodiment, buffer exchange and concentration and/or sterilization and filtration are performed on the solution containing the mRNA molecules obtained in step (g).

In one embodiment, the step of buffer exchange and concentration is performed by using a LiCl method and/or a magnetic particle method.

In one embodiment, the magnetic particle method comprises repeating the steps (c)-(e).

In any embodiment of the present disclosure, the DNA template may be a single-stranded or double-stranded linear or circular DNA. In a preferred embodiment, the DNA template is linearized prior to in vitro transcription. In a preferred embodiment, the DNA template is a linearized plasmid.

In preferred embodiments of the present disclosure, the magnetic particles are a carboxyl magnetic beads, such as Dynabeads^{™} MyOne^{™} carboxylic acid magnetic beads.

In preferred embodiments of the present disclosure, the positive-pressure affinity chromatography comprises Oligo(dT)₂₅ affinity packing, such as POROS^{™} Oligo(dT)₂₅ affinity packing.

In any embodiment of the present disclosure, the method is performed in an automated, semi-automated, or manual manner.

Further, the present disclosure relates to a system for separating mRNA molecules, comprising:
a first purification module comprising magnetic particles, wherein the magnetic particles comprise ligands capable of binding to the mRNA molecules;
a second purification module comprising an affinity chromatography column, wherein the affinity chromatography column is configured to operate in a positive-pressure mode; and
a fluid module comprising a container for containing a solution containing the mRNA, wherein the fluid module is capable of fluid communication with the first purification module and the second purification module.

In one embodiment, the system further comprises a feed module configured to provide a source of the mRNA molecules, and/or a collection module configured to collect the mRNA molecules. In one embodiment, the feed module comprises a container for containing an IVT reaction reagent. In another embodiment, the feed module comprises a container for IVT reaction.

In one embodiment, the fluid module further comprises a liquid transfer device. In one embodiment, the liquid transfer device is any device or means capable of transferring liquid, such as a pipettor, such as a manual or automatic pipettor, a pipette, a pipetting workstation, and the like.

In one embodiment, the fluid module further comprises a conduit that facilitates fluid communication of the first purification module, the second purification module, and the fluid module, and optionally the feed module and the collection module.

In one embodiment, the first purification module comprises a magnetic separator. In another embodiment, the first purification module comprises a magnetic field.

In one embodiment, the fluid module further comprises a container for containing an elution solution for eluting the mRNA from the magnetic particles, and a container for containing an elution solution for eluting the mRNA from the affinity chromatography column.

In one embodiment, the fluid module further comprises a container for containing a wash solution for washing the magnetic particles, and a container for containing a wash solution for washing the affinity chromatography column.

In one embodiment, the system further comprises one or more additional modules including, but not limited to, one or more devices configured to perform the following operations:
performing in vitro transcription on a DNA template to generate a solution containing the mRNA molecules;
capping and/or tailing the mRNA molecules;
performing buffer exchange and concentration on a solution containing the mRNA molecules; and/or
performing sterilization and filtration on a solution containing the mRNA molecules.

In one embodiment, the buffer exchange and concentration is performed by using a LiCl method. In one embodiment, the buffer exchange and concentration is performed by using a magnetic particle method. In one embodiment, the additional module for performing buffer exchange and concentration is the same as the first purification module. In one embodiment, the additional module for performing buffer exchange and concentration is different from the first purification module.

In one embodiment, the system is configured to operate in an automated, semi-automated, or manual manner. Therefore, in one embodiment, the system further comprises a control module configured to control various modules of the system to operate in an automated or semi-automated manner.

In some embodiments of the method or system of the present disclosure, the mRNA molecules comprise one or more functional nucleotide analogs. Preferably, the functional nucleotide analogs are one or more selected from: pseudouridine, 1-methyl-pseudouridine and 5-methylcytosine.

### BRIEF DESCRIPTION OF THE DRAWINGS

FIG. 1 shows two exemplary flowcharts (A and B) of a method for separating mRNA molecules according to the present invention.
FIG. 2 shows the comparison of purification results obtained by LiCl method and magnetic particle method. A, comparative experimental protocol of two purification methods; B, purity results by using the two methods of purification after IVT reaction (before capping); C, mRNA purity and capping rate results of the two purification methods after IVT.
FIG. 3 shows results of analysis of impurities introduced by magnetic bead method. A, IFN-β analysis results of mRNA stock solution in BJ cells; B, results of activity analysis of mRNA stock solution in BJ cells; C, analysis results of cytotoxicity of magnetic beads in BC2C12 mice muscle cells.
FIG. 4 shows the results of hematological and blood biochemical analysis of mice injected with magnetic beads via tail vein.
FIG. 5 shows the yield results of separating mRNA molecules by negative-pressure affinity chromatography. NC: positive-pressure affinity chromatography.
FIG. 6 shows a flowchart of multiple methods for separating mRNA molecules, wherein Procedure 3 exemplifies a method according to the present invention.
FIG. 7 shows a comparison of FA purity analysis results for Procedure 1, Procedure 2, and Procedure 3.
FIG. 8 shows a comparison of capping rate results for Procedure 1, Procedure 2, and Procedure 3.
FIG. 9 shows a comparison of protein residue detection results for Procedure 1, Procedure 2, and Procedure 3.
FIG. 10 shows a comparison of in vitro expression results for Procedure 1, Procedure 2, and Procedure 3.
FIG. 11 shows a comparison of protein residue detection results for Procedure 1 and Procedure 4.
FIG. 12 shows a comparison of mRNA purity, capping rate, DNA residue, dsRNA residue, and in vitro protein expression results for Procedure 3 and Procedure 5.
FIG. 13 shows a flowchart of a method for separating mRNA molecules, wherein Procedure 7 exemplifies a method according to the present invention.
FIG. 14 shows a comparison of FA purity analysis results for Procedure 6 and Procedure 7.
FIG. 15 shows a comparison of capping rate results for Procedure 6 and Procedure 7.
FIG. 16 shows a comparison of DNA residue detection results for Procedure 6 and Procedure 7.
FIG. 17 shows a comparison of dsRNA residue detection results for Procedure 6 and Procedure 7.
FIG. 18 shows a comparison of results of immunologic factor IFN-β detection in BJ fibroblasts for Procedure 6 and Procedure 7.
FIG. 19 shows a comparison of results of IFN-β content detected in supernatants from transfected BJ cells using IFN-α/β Reporter HEK 293 cells for Procedure 6 and Procedure 7.
FIGs. 20A-20E show a comparison of results of in vitro protein expression assay in A549 cells for Procedure 6 and Procedure 7.

### DETAILED DESCRIPTION OF EMBODIMENTS

Specific embodiments of the present invention will be described in detail below.

Unless otherwise defined, all technical and scientific terms used herein have the same meaning as commonly understood by one of ordinary skill in the art to which this invention belongs. The following references provide those skilled in the art with a general definition of many of the terms used in this invention: Dictionary of Biochemistry and Molecular Biology, (2nd Edition) J. Stenesh (ed.), Wiley - Interscience (1989); Dictionary of Microbiology and Molecular Biology (3rd Edition), P. Singleton and D. Sainsbury (ed.), Wiley - Interscience (2007); Chambers Dictionary of Science and Technology (2nd Edition), P. Walker (ed.), Chambers (2007); Glossary of Genetics (5th Edition), R. Rieger et al. (eds.), Springer - Verlag (1991); and The HarperCollins Dictionary of Biology, W. G. Hale and J. P. Margham (ed.), HarperCollins (1991).

As used herein, the term "a" or "an" does not indicate a limitation of quantity, but rather indicates the presence of at least one referenced item. The term "or" is not intended to be exclusive but rather indicate the presence of at least one of the referenced items (e.g., components), and include cases where a combination of the referenced items may exist. As used herein, the terms "comprising," "including," "having," or "containing" and similar expressions used indicate that other items, in addition to the items listed thereafter and their equivalents, may also fall within the scope.

Approximate terms in the present application are used to modify quantities, meaning that the present invention is not limited to the stated quantities, but also includes acceptable modifications close to the stated quantities that do not result in change to the relevant essential functions. Accordingly, a numerical value modified by "approximately", "about" and "around", and the like, means that the present invention is not limited to the exact numerical value. In some embodiments, the approximate terms may correspond to the accuracy of the instrument measuring the numerical value. The numerical ranges in the present invention may be combined and/or interchanged, and unless clearly stated otherwise, the numerical ranges include sub-ranges of all numerical values encompassed therein.

The term "messenger ribonucleic acid", also known as "messenger RNA" or "mRNA", is a type of single-stranded ribonucleic acid that is transcribed from one strand of DNA as a template, carries genetic information, and can guide the synthesis of proteins. The mRNA vaccine involves introducing mRNA encoding the antigen protein into the human body, where the mRNA is directly translated to form corresponding antigenic proteins, thereby inducing a specific immune response in the body to achieve the effect of preventive immunity. An RNA molecule, an mRNA molecule, or an mRNA vaccine herein has the same meaning unless indicated to the contrary. In some embodiments, the "messenger ribonucleic acid", "messenger RNA" or "mRNA" comprises a functional nucleotide analog. In some embodiments, the functional nucleotide analog comprises a non-standard nucleobase. In some embodiments, a standard nucleobase (e.g., adenine, guanine, uracil, thymine, and cytosine) in a nucleotide can be modified or replaced to provide one or more functional analogs of the nucleotide. Exemplary modifications of a nucleobase, includes, but are not limited to, one or more substitutions or modifications, including, but not limited to, alkyl, aryl, halogen, oxo, hydroxy, alkoxy, and/or thio substitutions; one or more fused or open ring, oxidation and/or reduction.

In some embodiments, the nucleic acid molecule of the present disclosure comprises an mRNA molecule. In particular embodiments, the nucleic acid molecule comprises at least one coding region (e.g., an open reading frame (ORF)) encoding a peptide or polypeptide of interest. In some embodiments, the nucleic acid molecule further comprises at least one untranslated region (UTR). In particular embodiments, the untranslated region (UTR) is located upstream (5' end) of the coding region and is referred to herein as 5'-UTR. In particular embodiments, the untranslated region (UTR) is located downstream (3' end) of the coding region and is referred to herein as 3'-UTR. In particular embodiments, the nucleic acid molecule comprises both a 5'-UTR and a 3'-UTR. In some embodiments, the 5'-UTR comprises a 5'-cap structure. In some embodiments, the nucleic acid molecule comprises a Kozak sequence (e.g., in 5'-UTR). In some embodiments, the nucleic acid molecule comprises a poly-A region (e.g., in a 3'-UTR). In some embodiments, the nucleic acid molecule comprises a polyadenylation signal (e.g., in 3'-UTR). In some embodiments, the nucleic acid molecule comprises a stabilizing region (e.g., in a 3'-UTR). In some embodiments, the nucleic acid molecule comprises a secondary structure. In some embodiments, the secondary structure is a stem-loop structure. In some embodiments, the nucleic acid molecule comprises a stem-loop sequence (e.g., in a 5'-UTR and/or a 3'-UTR). In some embodiments, the nucleic acid molecule comprises one or more intron regions that can be excised during splicing. In particular embodiments, the nucleic acid molecule comprises one or more regions selected from a 5'-UTR and a coding region. In particular embodiments, the nucleic acid molecule comprises one or more regions selected from a coding region and a 3'-UTR. In particular embodiments, the nucleic acid molecule comprises one or more regions selected from a 5'-UTR, a coding region, and a 3'-UTR.

In some embodiments, the non-standard nucleobase is a modified uracil. Exemplary nucleobases and nucleosides with a modified uracil include pseudouridine (ψ), pyridin-4-one ribonucleoside, 5-aza-uracil, 6-aza-uracil, 2-thio-5-aza-uracil, 2-thiouracil (s2U), 4-thio-uracil (s4U), 4-thio-pseudouridine, 2-thio-pseudouridine, 5-hydroxy-uracil (ho5U), 5-aminoallyl-uracil, 5-halo-uracil (for example, 5-iodo-uracil or 5-bromo-uracil), 3-methyluracil (m3U), 5-methyluracil (mo5U), uracil 5-oxoacetic acid (cmo5U), uracil 5-oxyacetic acid methyl ester (mcmo5U), 5-carboxymethyl-uracil (cm5U), 1-carboxymethyl-pseudouridine, 5-carboxyhydroxymethyl-uracil (chm5U), 5-carboxymethyl-uracilmethyl ester (mchm5U), 5-methoxycarbonylmethyluracil (mcm5U), 5-methoxycarbonylmethyl-2-thiouracil (mcm5s2U), 5-aminomethyl-2-thiouracil (nm5s2U), 5-methylaminomethyl-2-uracil (mnm5U), 5-methylaminomethyl-2-thiouracil (mnm5s2U), 5-methylaminomethyl-2-selenouracil (mnm5se2U), 5-carbamoylmethyluracil (ncm5U), 5-carboxymethylaminomethyluracil (cmnm5U), 5-carboxymethylaminomethyl-2-thiouracil (cmnm5s2U), 5-propynyluracil, 1-propynyl-pseudouracil, 5-taurinemethyluracil (τm5U), 1-taurinemethyl-pseudouridine, 5-taurinemethyl-2-thiouridine (τm5s2U), 1-taurinemethyl-4-thio-pseudouridine, 5-methyl-uracil (m5U, which has a nucleobase deoxythymidine), 1-methyl-pseudouridine (m1ψ), 1-ethyl-pseudoneuraminide (Et1ψ), and 5-methyl-2-thio-uracil (m5s2U). In some embodiments, the non-standard nucleobase of functional nucleotide analogs may independently be purine, pyrimidine, purine, or pyrimidine analogs. For example, in some embodiments, the non-canonical nucleobase may be a modified adenine, cytosine, guanine, uracil, or hypoxanthine. In other embodiments, the non-canonical nucleobases may also include, for example, naturally occurring and synthetic derivatives of bases, including pyrazolo[3, 4-d]pyrimidine, 5-methylcytosine (5-me-C), 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-propynyluracil and cytosine, 6-azouracil, cytosine and thymine, 5-uracil (pseudouracil), 4-thiouracil, 8-halo (for example, 8-bromo), 8-amino, 8-thiol, 8-thioalkyl, 8-hydroxy and other 8-substituted adenines and guanines, 5-halo (especially 5-bromo, 5-trifluoromethyl and other 5-substituted uracils and cytosines), 7-methylguanine and 7-methyladenine, 8-azaguanine and 8-azaadenine, deazaguanine, 7-deazaguanine, 3-deazaguanine, deazaadenine, 7-deazaadenine, 3-deazaadenine, pyrazolo[3,4-d]pyrimidine, imidazo[1,5-a][1,3,5]triazinone, 9-deazapurine, imidazo[4,5-d]pyrazine, thiazolo[4,5-d]pyrimidine, pyrazin-2-one, 1,2,4-triazine, pyridazine and 1,3,5-triazine.

As described herein, 0% to 100% of all nucleotides of one type (e.g., in the payload nucleic acid molecule, all purine-containing nucleotides of one type, or all pyrimidine-containing nucleotides of one type, or all A, G, C, T, or U) may be the functional nucleotide analogs described herein. For example, in various embodiments, about 1% to about 20%, from about 1% to about 25%, from about 1% to about 50%, from about 1% to about 60%, from about 1% to about 70%, from about 1% to about 80%, from about 1% to about 90%, from about 1% to about 95%, from about 10% to about 20%, from about 10% to about 25%, from about 10% to about 50%, from about 10% to about 60%, from about 10% to about 70%, from about 10% to about 80%, from about 10% to about 90%, from about 10% to about 95%, from about 10% to about 100%, from about 20% to about 25%, from about 20% to about 50%, from about 20% to about 60%, from about 20% to about 70%, from about 20% to about 80%, from about 20% to about 90%, from about 20% to about 95%, from about 20% to about 100%, from about 50% to about 60%, from about 50% to about 70%, from about 50% to about 80%, from about 50% to about 90%, from about 50% to about 95%, from about 50% to about 100%, from about 70% to about 80%, from about 70% to about 90%, from about 70% to about 95%, from about 70% to about 100%, from about 80% to about 90%, from about 80% to about 95%, from about 80% to about 100%, from about 90% to about 95%, from about 90% to about 100%, or from about 95% to about 100% are the functional nucleotide analogs described herein. In any one of these embodiments, the functional nucleotide analogs may be present at any position of a nucleic acid molecule, including 5' end, 3' end, and/or one or more internal positions. In some embodiments, a single nucleic acid molecule may comprise different sugar modifications, different nucleobase modifications, and/or different types of nucleoside bonds (e.g., backbone structure).

The term "separated" or "purified" refers to a substance that is essentially or substantially free of components normally present with the substance in its natural or initial state. Therefore, the separated mRNA molecules according to the present disclosure are preferably free of substances normally associated with the mRNA molecules in their natural or initial environment. A compound or entity is considered to be separated or purified when it is removed from essentially all other compounds or entities, i.e. preferably at least about 90%, more preferably at least about 91%, 92%, 93%, 94%, 95%, 96%, 97%, 98%, 99%, or greater than 99% pure. In some embodiments, the mRNA molecules separated by the methods of the present disclosure comprise less than 10%, less than 9%, less than 8%, less than 7%, less than 6%, less than 5%, less than 4%, less than 3%, less than 2%, less than 1%, less than 0.5%, less than 0.1% of impurities other than the full-length mRNA molecules. The impurities include IVT contaminants, such as proteins, enzymes, free nucleotides, and/or short polymers. In some embodiments, the mRNA molecules prepared according to the present invention are essentially free of short polymers or null transcripts.

Full-length or null transcripts of mRNA can be detected and quantified using any method known in the art. In some embodiments, the separated mRNA molecules are detected using blotting, capillary electrophoresis, chromatography, fluorescence, gel electrophoresis, HPLC, silver staining, spectroscopy, ultraviolet (UV), or UPLC, or a combination thereof. Other detection methods known in the art are included in the present invention. In some embodiments, mRNA molecules are detected by capillary electrophoresis separation using UV absorption spectroscopy. In some embodiments, the mRNA is denatured with a glyoxal dye prior to gel electrophoresis ("glyoxal gel electrophoresis"). In some embodiments, a synthetic mRNA is characterized prior to capping or tailing. In some embodiments, a synthetic mRNA is characterized after capping and tailing.

The term "plasmid" refers to a genetic construct consisting of genetic material (i.e. nucleic acid). Typically, a plasmid contains an origin of replication that is functional in a bacterial host cell, such as Escherichia coli, and a selectable marker for detecting a bacterial host cell comprising the plasmid. In the present disclosure, a plasmid may comprise one or more genetic elements configured such that the inserted coding sequence can be transcribed and translated in a suitable expression cell. Additionally, the plasmid may comprise one or more nucleic acid segments, genes, promoters, enhancers, activators, polycloning sites, or any combination thereof, including segments obtained or derived from one or more natural and/or artificial sources.

The term "LiCl method", also referred to as "LiCl precipitation "refers to a method that employs a high concentration of lithium cations to selectively precipitate RNA. Typically, the synthetic RNA is added to a high-concentration LiCl solution and incubated at -20°C, followed by high-speed centrifugation at 4°C, such as 12,000-15,000 g, to precipitate mRNA for purification. The LiCl method is a conventional method in the art for purifying mRNA.

The term "magnetic particles" are defined herein as particles that can be attracted by a magnetic field. Meanwhile, the magnetic particles used in the method of the present disclosure should not aggregate in the absence of a magnetic field. In other words, the behavior of the magnetic particles should be similar to superparamagnetic particles. The magnetic particles may have any symmetrical shape, such as spherical or cubic shape, or any asymmetrical shape. Spherical magnetic particles are often referred to as magnetic beads. It should be understood that the terms "magnetic particles" and "magnetic beads" are used interchangeably herein without limiting the scope to a magnetic particles having spherical shapes. The volume-weighted median diameter (d50, v) of magnetic particles suitable for use in the method of the present disclosure may be in the range of 10 nm to 100 µm, for example 10 nm, 20 nm, 100 nm, 1 µm, 2 µm, 10 µm, 15 µm, 20 µm, 25 µm, 30 µm, 35 µm, 50 µm, 60 µm, 70 µm, 80 µm, 90 µm, 95 µm or 100 µm. Preferably, the volume-weighted median diameter (d50, v) of magnetic particles or the particle size of magnetic beads is 20 nm-5 µm. Typically, the smaller the diameter of a magnetic particle, the larger the overall contact surface area. It should be understood that the diameter of a magnetic particle is not limited to this range, depending on factors such as the category, concentration, volume, and separation system of the biomolecule to be separated.

The magnetic particles comprise ligands capable of binding to biomolecules. The ligands may be covalently coupled to the surface or interior of the magnetic particles. It should be understood that the type of a ligand and its affinity constants k_{off}/kₒₙ for the biomolecule are selected based on the type of biomolecule to be separated. In one embodiment, the ligand is a carboxyl group. Furthermore, it should be understood that the concentration of one or more ligands of each magnetic particle depends on the concentration of the biomolecules to be separated, the size of magnetic particles, and/or the total volume of magnetic particles added to the magnetic separator. In preferred embodiments of the present disclosure, the magnetic particles used in the method for separating mRNA molecules are carboxyl magnetic beads.

As used herein, the terms "magnetic bead method" and "magnetic particle method" are used interchangeably to refer to a method of purifying biomolecules, such as mRNA molecules, by using the magnetic particles or the magnetic beads.

The term "magnetic separator" has its conventional meaning in the field of separation methods and refers to a device for separating magnetic particles from fluid. In preferred embodiments of the present disclosure, the magnetic separator used in the method for separating a biomolecule is a high-gradient magnetic separator, or referred to as a high-gradient magnetic separation system (HGMS). The magnetic separator is configured to separate magnetic particles with bound mRNA molecules from the rest of feed. The magnetic separator contains a component of magnetic or magnetizable material internally that attracts magnetic particles when a magnetic field is applied.

In one embodiment, the magnetic bead method further comprises further filtering the fluid with a 0.22 µm membrane after separating the magnetic particles from the fluid.

The term "affinity chromatography" is a chromatographic method that utilizes the binding properties of a stationary phase to separate molecules. Molecules that have a certain binding ability to the substance to be separated are attached to the affinity chromatography packing, and their binding is reversible, and under altered mobile phase conditions, the two can be separated. The most prominent structural feature of mRNA in eukaryotic cells is that it has 5'-end cap and 3'-end Poly(A) tail. This structure provides an extremely convenient selection marker for purification of eukaryotic mRNA. Affinity chromatography for separating mRNA typically utilizes the complementarity between oligo(dT) and the Poly(A) tail to capture mRNA. In preferred embodiments of the present disclosure, affinity chromatography used in the method for separating mRNA molecules utilizes Oligo(dT)₂₅ affinity packing.

In one embodiment, the affinity chromatography used in the method for separating mRNA molecules is performed in a positive-pressure manner. The so-called " positive pressure" or "negative pressure" refers to the pressure state within the closed container of a chromatography system. When the pressure in a closed container is greater than atmospheric pressure, it is called positive pressure, while when the pressure is less than atmospheric pressure, it is called negative pressure. Positive pressure is the use of pressurized gas (compressed air or nitrogen) to cause the solution to enter the solid phase extraction column at a controlled flow rate for related operations; negative pressure is the use of a pump to extract air from the instrument and causing the solution to flow through the solid phase extraction column for related operations.

The term "in vitro transcription ", also called "IVT", refers to the process of generating RNA by using DNA as a template in an in vitro cell-free system, mimicking the in vivo transcription process. During In vitro transcription, DNA containing T7, T3, or SP6 promoter sequences is typically used as a template to synthesize RNA complementary to one strand of the template DNA using NTPs or modified bases as substrates with T7, T3, or SP6 RNA polymerase, respectively. This allows for the large-scale generation of RNA molecules in a cell-free environment. Prior to in vitro transcription, the DNA template is usually obtained through processes such as 1) amplification of DNA via Escherichia coli fermentation, DNA purification, and linearization of plasmid DNA, or 2) enzymatic amplification and purification of DNA transcription region fragments.

The resulting mRNA molecules may optionally be subjected to 5'-end capping and/or 3'-end tailing during or after in vitro transcription. The presence of a "cap" may provide resistance to nucleases found in most eukaryotic cells. The presence of a "tail" can protect mRNA from exonuclease degradation and/or modulate protein expression levels. Capping and tailing techniques are well known to those skilled in the art.

The term "cap" refers to a structure comprising or consisting essentially of a 5'-end nucleoside-5'-triphosphate, which typically binds to an uncapped RNA (e.g., an uncapped RNA having a 5'-diphosphate). In some embodiments, the cap is or comprises a guanine nucleotide. In some embodiments, the cap is or comprises a naturally occurring RNA 5'-cap, including, for example, but not limited to, an N7-methylguanosine cap having a structure designated "m7G". In some embodiments, the cap is or comprises a synthetic cap analog that is similar to an RNA cap structure and has the ability to stabilize RNA if attached thereto, including, for example, but not limited to, anti-reverse cap analogs (ARCAs) known in the art. Those skilled in the art will appreciate that methods for binding the cap to the 5' end of RNA are known in the art. For example, in some embodiments, capped RNA can be obtained by in vitro capping RNA having a 5' triphosphate group or RNA having a 5' diphosphate group with a capping enzyme system, including, for example, but not limited to, a vaccinia capping enzyme system or a Saccharomyces cerevisiae capping enzyme system. Alternatively, capped RNA can be obtained by in vitro transcription (IVT) of a DNA template, wherein the IVT system contains, in addition to GTP, a cap analog, for example, as known in the art. Non-limiting examples of cap analogs include m7GpppG cap analogs or N7-methyl-, 2'-O-methyl-GpppG ARCA cap analogs or N7-methyl-, 3'-O-methyl-GpppG ARCA cap analogs or any commercially available cap analogs including, for example, CleanCap (Trilink), EZ Cap, and the like. In some embodiments, the cap analog is or comprises a trinucleotide cap analog. Various cap analogs are described herein and are known in the art, for example, commercially available.

The 5'-end cap can be added as follows: first, one terminal phosphate group is removed from the 5' nucleotide by an RNA terminal phosphatase, leaving two terminal phosphate groups; guanosine triphosphate (GTP) is then added to the terminal phosphate group by a guanylyltransferase, creating a 5,5,5 triphosphate bond; subsequently the 7-nitrogen of guanine is methylated using methyltransferase. Examples of cap structures include, but are not limited to, m7G(5')ppp(5')A, G(5')ppp(5')A and G(5')ppp(5')G. More cap structures have been described in the prior art, such as U.S. Patent Application No. US2016/0032356, Ashiqul Haque et al., Chemically modified hCFTR mRNAs recuperate lung function in a mouse model of cystic fibrosis, Scientific Reports (2018)8:16776, and Kore et al., Recent Developments in 5'-Terminal Cap Analogs: Synthesisand Biological Ramifications, Mini-Reviews in Organic Chemistry, 2008, 5, 179-192, which is incorporated herein by reference.

The tail structure may comprise a Poly(A) tail. 10, 20, 30, 40, 50, 60, 70, 80, 90, 100, 150, 200, 300, 500 or more nucleotides at the 3'-end of mRNA may be a Poly(A) tail. The Poly(A) tail may comprise adenosine nucleotides of at least 50%, 55%, 65%, 70%, 75%, 80%, 85%, 90%, 92%, 94%, 95%, 96%, 97%, 98%, or 99% or 100%.

In some embodiments, the mRNA is purified before capping and tailing. In some embodiments, the mRNA is purified after capping and tailing. In some embodiments, the mRNA is purified both before and after capping and tailing. In some embodiments, the mRNA is purified by the LiCl method before or after capping and tailing, or both before and after capping and tailing. In some embodiments, the mRNA is purified by a magnetic particle method before or after capping and tailing, or both before and after capping and tailing. In some embodiments, the mRNA is purified by affinity chromatography before or after capping and tailing, or both before and after capping and tailing.

In some embodiments, capping and/or tailing is completed concomitantly with transcription. Therefore, the mRNA can be purified directly after the IVT step is completed. In some embodiments, the mRNA molecules are purified before and/or after tailing. In some embodiments, the mRNA molecules are purified before and/or after capping.

The term "module" refers to a hardware or software unit consisting of one or more basic functional units, which may also be referred to as a device or program. Modules are typically named after their function or use, including any hardware or software necessary to achieve the desired functionality. For example, the purification module consists of one or more devices for achieving purification purposes, while the control module contains a control program and hardware configured to execute the program, such as a processor, a storage, a memory, a magnetic disk, and the like. It should be understood that the meaning and components of "module" are well known to those skilled in the art, and that the various modules used in the methods and systems of the present invention will be readily configured upon reading the present disclosure. Exemplary configurations of modules are described in more detail below.

The term "automation" refers to the process of operating a device in a production operation in a manner of automatic control and automatic adjustment according to a predetermined pattern. As the name suggests, the term "semi-automated" refers to an automated mode in which the work cycle is automatically carried out with human intervention. The automated, semi-automated or manual methods are selected based on a comprehensive consideration of operating conditions, cost-benefit ratio and the like, and are all within the contemplated scope of the present invention. For example, with the methods and systems of the present invention, 168 mRNA samples (mg level) can be processed in 24 h in an automated manner, whereas 24 mRNA samples (mg level) can be processed by one person in 5 days. In a preferred embodiment, the method or system according to the present invention is configured to operate in an automated manner.

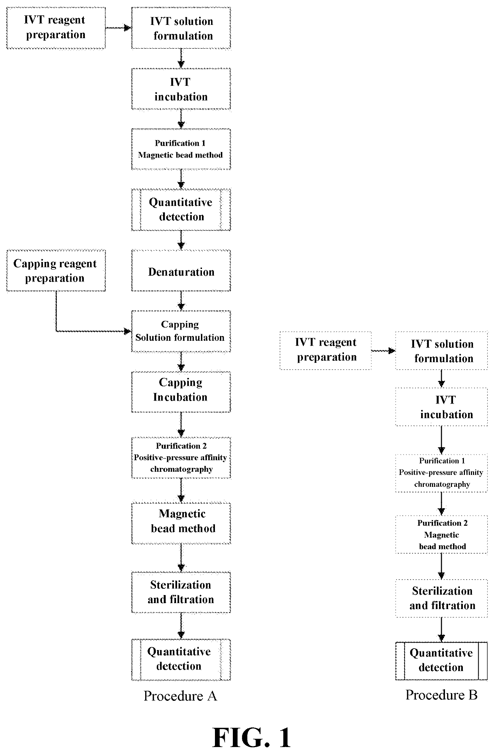

FIG. 1 Procedure A shows an exemplary flowchart of a method for separating mRNA molecules according to the present invention. First, an in vitro transcription (IVT) reaction is performed on the linearized DNA template. The transcription template is usually obtained through processes such as 1) amplification of DNA via escherichia coli fermentation, DNA purification, and linearization of plasmid DNA, or 2) enzymatic amplification and purification of DNA transcription region fragments. In addition to the DNA template, the in vitro transcription system further comprises nucleoside triphosphate (NTP) substrates, RNA polymerases, inorganic pyrophosphatases, RNase inhibitors and other reagents. Optionally, the IVT reagent further comprises one or more of a 5'-end cap analog, a capping enzyme, a Poly(A) polymerase, a DNase, proteinase K, and the like. The 5' capping and/or 3' tailing can be performed in the IVT reaction or as a separate step after transcription. DNA residue needs to be strictly controlled during mRNA production. DNase treatment can hydrolyze the DNA template, and proteinase K treatment can degrade the protein, thereby facilitating subsequent purification. Depending on the reaction volume, IVT can be performed in any suitable container, such as a 24-well plate, a 96-well plate, a microcentrifuge tube, and the like.

After IVT incubation reaction, primary purification is performed by a magnetic bead method. The magnetic beads containing ligands capable of binding to mRNA molecules are provided, preferably carboxyl magnetic beads. The magnetic bead binds to mRNA by means of a salt bridge, and adsorbs RNA by forming an ion bridge of an RNA-salt ion-carboxyl group in a purification buffer system containing PEG or high salt ions. This binding is reversible, and the ionic bridge is broken in TE buffer free of PEG and salt ions, thereby eluting RNA molecule.

The solution after IVT incubation is contacted with the magnetic beads to obtain magnetic beads containing bound mRNA molecules, and the magnetic beads are separated using a magnetic field; the mRNA molecules are eluted from the separated magnetic beads, resulting in a primary purified mRNA solution.

After IVT reaction, the primary purified mRNA solution is quantified. Any method known in the art can be used to detect and quantify mRNA molecules. Preferably, a commercially available RNA quantification kit, such as the Agilent RNA 6000 Nano kit (# 5067-1511), is used. Based on the quantitative results, capping and/or tailing solutions are prepared and further incubated. After this step, the full-length product of mRNA is obtained, including the 5' cap structure and the 3' poly(A) tail.

Next, secondary purification is performed by affinity chromatography. Given that intact mRNA contains a 3' Poly(A) tail, mRNA can be captured and purified by using an affinity packing material with oligo(dT) as a ligand (e.g.,POROS^{™} Oligo(dT)₂₅affinitypacking material) through complementary pairing with the Poly(A) tail. In this step, affinity chromatography is performed in a positive-pressure manner, eluting mRNA molecules to obtain a secondary purified mRNA solution.

If IVT employs a co-transcription mode, i.e. transcription directly yields an mRNA full-length product including the 5' cap structure and 3' poly(A) tail, the mRNA full-length transcript can be directly obtained through primary purification after the IVT incubation reaction, followed by secondary purification via affinity chromatography.

At this time, the mRNA solution has reached a relatively high purity, and subjected to buffer exchange and concentration, which is mainly intended to adjust the mRNA concentration in the stock solution and replace the buffer system. Buffer exchange and concentration can be performed by either the LiCl method or the magnetic bead method. For the magnetic bead method, the same or different systems or devices as the primary purification can be used. To save on equipment or procedures, it is preferable to use the same magnetic bead separation device as the primary purification.

The mRNA after buffer exchange and concentration is formulated into a stock solution suitable for cryopreservation, followed by sterilization and filtration and aseptic dispensing into containers suitable for cryopreservation.

FIG. 1 Procedure B shows another exemplary flowchart of a method for separating mRNA molecules according to the present invention. First, an in vitro transcription (IVT) reaction is performed on the linearized DNA template as previously described. 5' capping and/or 3' tailing can be performed in an IVT reaction in a co-transcription manner, or as a separate step after the IVT reaction.

After IVT incubation reaction, or optionally after capping and/or tailing, the full-length product of mRNA, including the 5' cap structure and 3'-poly(A) tail, is obtained.

In one embodiment, optionally after obtaining the full-length product of mRNA, the mRNA solution is subjected to capping.

The full-length product of mRNA is primarily purified by affinity chromatography. Given that intact mRNA contains a 3' Poly(A) tail, mRNA can be captured and purified by using an affinity packing material with oligo(dT) as a ligand (e.g.,POROS^{™} Oligo(dT)₂₅affinitypacking material) through complementary pairing with the Poly(A) tail. In this step, affinity chromatography is performed in a positive-pressure manner, eluting mRNA molecules to obtain a primarily purified mRNA solution.

In one embodiment, the primary purified mRNA solution is subjected to capping, optionally after affinity chromatography.

Next, secondary purification is performed by the magnetic bead method. The magnetic beads containing ligands capable of binding to mRNA molecules are provided, preferably carboxyl magnetic beads. The magnetic bead binds to mRNA by means of a salt bridge, and adsorbs RNA by forming an ion bridge of an RNA-salt ion-carboxyl group in a purification buffer system containing PEG or high salt ions. This binding is reversible, and the ionic bridge is broken in TE buffer free of PEG and salt ions, thereby eluting RNA molecule.

The mRNA solution after primary purification is contacted with the magnetic beads to obtain magnetic beads containing bound mRNA molecules, and the magnetic beads are separated using a magnetic field; the mRNA molecules are eluted from the separated magnetic beads, resulting in a secondary purified mRNA solution.

In one embodiment, the secondary purified mRNA solution is subjected to capping, optionally after purification via magnetic bead method.

Buffer exchange and concentration are performed simultaneously with secondary purification via magnetic bead method, primarily aiming to adjust the mRNA concentration in the stock solution and replace the buffer system. Alternatively, buffer exchange and concentration of the secondary purified mRNA solution are performed in a separation step, such as through LiCl method or magnetic bead method. For the magnetic bead method, the same or different systems or devices as the primary purification can be used. To save on equipment or procedures, it is preferable to use the same magnetic bead separation device as the primary purification.

The mRNA after buffer exchange and concentration is formulated into a stock solution suitable for cryopreservation, followed by sterilization and filtration and aseptic dispensing into containers suitable for cryopreservation.

In any embodiment of the present invention, optionally prior to capping the mRNA in solution, purification/buffer exchange and concentration are performed via the LiCl method or magnetic bead method.

It should be understood that the procedures and embodiments described above are merely exemplary. Not all procedures and devices are mandatory for the methods and systems of the present invention. The scope of protection of the present invention is defined by the appended claims. The following examples are included to provide additional guidance to those of ordinary skill in the art in practicing the claimed invention. These examples do not limit the invention as defined in the appended claims.

### EXAMPLES

### Example 1: Experimental materials and methods

### 1.1 DNA linearization

The DNA plasmid template was linearized using restriction enzyme digestion. Each 10 µg of plasmid was mixed with 10 U of Esp3I/BsmB I and incubated at 37°C for 4 hours to ensure complete linearization. The reaction was stopped by adding 1/10 volume of 3 M sodium acetate (pH 5.5) and 2.5 volumes of ethanol, mixing thoroughly and cooling at -20°C for 1 h. The linearized DNA was precipitated by centrifugation at 13,800 g for 15 min at 4°C, washed twice with 70% ethanol, and resuspended in nuclease-free water.

### 1.2 IVT

### System of 20 µL of reaction mixture:

| | |
|---|---|
| RNase inhibitor (40 U/µL) | 0.5 µL |
| 10 × IVT reaction buffer | 2 µL |
| rNTP mixture (100 mM each) | 8 µL (final 10 mM each) |
| 1 M MgCl₂ | 0.8 µL |
| 0.1 M DTT | 2 µL |
| 100 U/mL inorganic pyrophosphatase | 0.8 µL |
| 100 mM NaCl | 1 µL |
| Linearized plasmid | 1 µg |
| T7 RNA polymerase mixture (KACTUS (China), # T7P-EE101) (50 kU/mL) | 1 µL |
| Nuclease-free H₂O | Supplement to 20 µL |

The reaction mixture was incubated at 37°C for 6 hours, then 1 µL of DNase I (no RNase, 1 U/µL) was added to remove the DNA template, and incubated at 37°C for 30 min.

### Capping IVT:

An in vitro transcription reactions was performed with T7 RNA polymerase using purified linear plasmids as a template. According to the instructions of HiScribe ^{®} T7 High Yield RNA Synthesis Kit (NEB, # E2040S), m7GpppAmpU or m7GpppAmpG were added for in vitro transcription, respectively, and mRNA having a Cap1 "cap" structure was synthesized.

### 1.3 LiCl method

The synthetic RNA was purified by adding a 0.5-fold volume of 7.5 M LiCl, 50 mM EDTA and incubating at -20°C for 45 min, followed by centrifugation at 13,800 g for 15 min at 4°C to precipitate the mRNA. The supernatant was then removed and the pellet was rinsed twice with pre-cooled 70% ethanol and the mRNA was resuspended in nuclease-free H₂O and stored at-20°C.

### 1.4 Capping

Each 10 µg aliquot of uncapped mRNA was heated at 65°C for 10 min, left on ice for 5 min, and then mixed with 10 U Vaccinia capping enzyme, 50 U mRNA Cap2'-O-methyltransferase, 0.2 mM SAM, 0.5 mM GTP, and 1 U RNase inhibitor and incubated at 37°C for 60 min to generate cap1 modification structure.

### 1.5 Affinity chromatography

Thermo fisher's POROS^{™} Oligo(dT)25 (# A47383) affinity packing was used to perform RNA purification by solid phase extraction instrument (in a positive or negative pressure manner). Equilibration/loading buffer : 10 mM Tris-HCl, 500 mM NaCl, 1 mM EDTA, PH 7.4. Wash buffer: 10 mM Tris-HCl, 100-300 mM NaCl, 1 mM EDTA, PH 7.4. Elution buffer: 10 mM Tris-HCl, 1 mM EDTA, PH 7.4 or directly water. For specific experimental procedures, refer to Thermo: POROS ^{™} Oligo(dT)25 Affnity Resin.

### 1.6 Quantitative detection

Aglient's Bioanalyzer RNA Kit with Reagents (Agilent RNA 6000 Nano Kit, #5067-1511) or Fragment Analyzer System RNA Kit (RNA Kit (15NT), #DNF-471-0500) were used.

### 1.7 Capping rate detection

The capping rate was detected using RNase H enzyme, streptavidin magnetic beads, and LC-MS. A biotin-TEG-labeled probe was designed at the 5' end of the mRNA. After RNase H enzyme cleavage and magnetic bead treatment, short 5' end nucleotide fragments with or without the cap structure at 5' end were obtained. LC-MS was employed for detection, and quantitative analysis of each component ratio was performed to determine the capping rate.

### 1.8 Magnetic bead purification

Purification was performed using Thermo fisher's Dynabeads^{™} MyOne^{™} carboxylic acid magnetic beads (#35401). The samples were bound to the magnetic beads, the magnetic beads were transferred using a magnetic stand, washed with 50%-80% ethanol, and finally eluted with nuclease-free water. For specific experimental operations, refer to the experimental protocol provided on the Thermo official website.

### 1.9 Physicochemical property testing

DNA residue detection was performed on mRNA samples using a plasmid DNA residue detection kit (PCR-fluorescent probe method) according to the protocol provided by the manufacturer. The total protein residue in the mRNA samples was detected using the Nano Orange^{™} Kit. The mRNA samples were tested for dsRNA residues using a Double-stranded-RNA (dsRNA) kit.

### 1.10 In vitro detection

### 1.10.1 In vitro IFN-β detection

BJ fibroblasts were purchased from ATCC. L-Glutamine and 10% heat-inactivated fetal bovine serum (Gibco-FBS) (Life Technologies, Thermofisher) were added to EMEM medium. Cells were seeded at 20,000 cells per well in a 96-well flat-bottom cell culture plate (Corning) and transfected after 24 hours. Cells were transfected with mRNA using Lipofectamine 2000 (Thermo Fisher Scientific). Cell culture supernatants were collected 48 hours after transfection and analyzed for IFN-β expression using an ELISA kit (R&D System).

1.10.2 IFN-α/β Reporter HEK 293 cell assay to detect IFN-β content in supernatant from transfected BJ cells

BJ fibroblasts were purchased from ATCC. L-Glutamine and 10% heat-inactivated fetal bovine serum (Gibco-FBS) (Life Technologies, Thermofisher) were added to EMEM medium. Cells were seeded at 20,000 cells per well in a 96-well flat-bottom cell culture plate (Corning) and transfected after 24 hours. Cells were transfected with mRNA using Lipofectamine 2000 (Thermo Fisher Scientific). Cell culture supernatants were harvested 48 hours after transfection.

IFN-α/β Reporter HEK 293 cells were purchased from Invivogen. L-Glutamine and 10% heat-inactivated fetal bovine serum (Gibco-FBS) (Life Technologies, Thermofisher) were added to DMEM medium. Cells were seeded at 20,000 cells per well in a 96-well flat-bottom cell culture plate (Corning) and cultured for 24 hours. The medium was discarded, and the supernatant sample from transfected BJ cells diluted with Opti-MEM was added, and the culture was continued. The supernatant was aspirated, QUANTI-Blue detection reagent (Invivogen) was added, and the absorbance value OD620 at 620 nm was detected using a microplate reader (MD). The IFN-β content in the supernatant sample from transfected BJ cells was calculated using a standard curve plotted from the standard concentration versus OD620.

### 1.10.3 Cell viability detection

BJ fibroblasts were purchased from ATCC. L-Glutamine and 10% heat-inactivated fetal bovine serum (Gibco-FBS) (Life Technologies, Thermofisher) were added to EMEM medium. Cells were seeded at 20,000 cells per well in a 96-well flat-bottom cell culture plate (Corning) and transfected after 24 hours. Cells were transfected with mRNA using Lipofectamine 2000 (Thermo Fisher Scientific). After 48 hours of transfection, 100 ul of Cell Titer-Glo^{®}Luminescent Cell Viability Assay reagent (Promega) was added, and the chemiluminescent signal was detected using a microplate reader (MD).

### 1.10.4 Cytotoxicity assay

C2C12 mouse muscle cells (C2C12) were purchased from ATCC. 10% heat-inactivated fetal bovine serum (Gibco-FBS) (Life Technologies, Thermofisher) was added to DMEM medium. Cells were seeded at 5,000-10,000 cells per well in a 96-well flat-bottom cell culture plate (Corning) and cultured for 24 hours. 50 ul of magnetic beads at different dilutions and magnetic beads containing Lipofectamine 2000 (Thermo Fisher Scientific) at different dilutions were added while cells without transfection were used as control. After 72 hours of culture, 100 ul of Cell Titer-Glo^{®} Luminescent cell viability reagent (Promega) was added, and the chemiluminescence signal was detected using a microplate reader (MD). Inhibition rate (%) = 100% * [(control well reading - experimental well reading)/(control well reading - blank well reading)].

### 1.10.5 In vitro expression assay - HEK293T analysis

eGFP: human embryonic kidney cells (HEK293T) were purchased from the Cell Bank of the Chinese Academy of Sciences, Shanghai. 10% fetal bovine serum (Gibco-FBS) (Life Technologies, Thermofisher) was added to DMEM medium. Cells were seeded at 20,000 cells per well in a 96-well flat-bottom cell culture plate (Corning) and transfected after 24 hours. Cells were transfected using Lipofectamine 2000 (Thermo Fisher Scientific) thoroughly mixed with mRNA encoding green fluorescent protein (eGFP). 24 hours after transfection, the expression level of eGFP protein was analyzed using a microplate reader.

Luc: human embryonic kidney cells (HEK293T) were purchased from the Cell Bank of the Chinese Academy of Sciences, Shanghai. 10% fetal bovine serum (Gibco-FBS) (Life Technologies, Thermofisher) was added to DMEM medium. Cells were seeded at 20,000 cells per well in a 96-well flat-bottom cell culture plate (Corning) and transfected after 24 hours. HEK293T cells were transfected using Lipofectamine 2000 (Thermo Fisher Scientific) thoroughly mixed with mRNA encoding luciferase (Luc). 24 hours after transfection, 100 ul of luciferase reporter gene detection reagent (Novozyme) was added and the expression level of Luc protein was analyzed using a microplate reader.

RBD: human embryonic kidney cells (HEK293T) were purchased from the Cell Bank of the Chinese Academy of Sciences, Shanghai. 10% heat-inactivated fetal bovine serum (Gibco-FBS) (Life Technologies, Thermofisher) was added to DMEM medium. Cells were seeded at 80,000 cells per well in a 6-well flat-bottom cell culture plate (Corning) and transfected after 24 hours. HEK293T cells were transfected using Lipofectamine 2000 (Thermo Fisher Scientific) thoroughly mixed with mRNA encoding the novel coronavirus spike protein receptor binding domain (S-RBD). 18-24 hours after transfection, the cell culture supernatant was collected, and the expression level of RBD protein was analyzed using an ELISA kit (Sino Biological).

H1N1 HA or H3N2 HA: human embryonic kidney cells (HEK293T) were purchased from the Cell Bank of the Chinese Academy of Sciences, Shanghai. 10% heat-inactivated fetal bovine serum (Gibco-FBS) (Life Technologies, Thermofisher) was added to DMEM medium. After cell growth reached ≥ 80% confluency, HEK293T cells were transfected with mRNA encoding influenza virus H1N1 HA or H3N2 HA protein using Lipofectamine Messenger MAX reagent (ThermoFisherScientific). Cell culture supernatants were collected 18 to 24 hours after transfection, treated with H1N1 HA primary antibody working solution (Sino Biological, # 11055-MM04T) or H3N2 HA primary antibody working solution (Sino Biological, # 11055-MM03) and secondary antibody working solution (Jackson immune Research, # 115-095-164), and then analyzed on a flow cytometer (BD, LSRFortessa).

### 1.10.6 In vitro expression assay - A549 analysis

eGFP: human non-small cell lung cancer cells (A549) were purchased from ATCC. 10% heat-inactivated fetal bovine serum (Gibco-FBS) (Life Technologies, Thermofisher) was added to F-12K medium (ATCC, 30-2004). Cells were seeded at 20,000 cells per well in a 96-well flat-bottom cell culture plate (Corning) and transfected after 24 hours. Cells were transfected using Lipofectamine 2000 (Thermo Fisher Scientific) thoroughly mixed with mRNA encoding green fluorescent protein (eGFP). 24 hours after transfection, the expression level of eGFP protein was analyzed using a microplate reader.

Luc: human non-small cell lung cancer cells (A549) were purchased from ATCC. 10% heat-inactivated fetal bovine serum (Gibco-FBS) (Life Technologies, Thermofisher) was added to F-12K medium (ATCC, 30-2004). Cells were seeded at 20,000 cells per well in a 96-well flat-bottom cell culture plate (Corning) and transfected after 24 hours. Cells were transfected using Lipofectamine 2000 (Thermo Fisher Scientific) thoroughly mixed with mRNA encoding luciferase (Luc). 24 hours after transfection, 100 ul of luciferase reporter gene detection reagent (Novozyme) was added and the expression level of Luc protein was analyzed using a microplate reader.

RBD: human non-small cell lung cancer cells (A549) were purchased from ATCC. 10% heat-inactivated fetal bovine serum (Gibco-FBS) (Life Technologies, Thermofisher) was added to F-12K medium (ATCC, 30-2004). Cells were seeded at 20,000 cells per well in a 96-well flat-bottom cell culture plate (Corning) and transfected after 24 hours. Cells were transfected using Lipofectamine 2000 (Thermo Fisher Scientific) thoroughly mixed with mRNA encoding the novel coronavirus spike protein receptor binding domain (S-RBD). 18-24 hours after transfection, the cell culture supernatant was collected, and the expression level of RBD protein was analyzed using an ELISA kit (Sino Biological).

RSV: human non-small cell lung cancer cells (A549) were purchased from ATCC. 10% heat-inactivated fetal bovine serum (Gibco-FBS) (Life Technologies, Thermofisher) was added to F-12K medium (ATCC, 30-2004). Cells were seeded at 35,000-40,000 cells per well in a 6-well flat-bottom cell culture plate (Corning) and transfected after 24 hours. Cells were transfected with mRNA using Lipofectamine 2000 (Thermo Fisher Scientific). Cells were collected 18-24 hours after transfection and analyzed by flow cytometry. The transfected cells were collected and added to 96-well plates, centrifuged, and then blocked with 2% BSA. Upon completion of blocking, pre-diluted antibody against F488 anti-respiratory syncytial virus fusion protein was used for incubation at 4°C for 1 hour. After incubation, the cells were washed, resuspended in DPBS, and subjected to flow cytometry analysis.

BA.4/5: human non-small cell lung cancer cells (A549) were purchased from ATCC. 10% heat-inactivated fetal bovine serum (Gibco-FBS) (Life Technologies, Thermofisher) was added to F-12K medium (ATCC, 30-2004). Cells were seeded at 20,000 cells per well in a 96-well flat-bottom cell culture plate (Corning) and transfected after 24 hours. Cells were transfected using Lipofectamine 2000 (Thermo Fisher Scientific) thoroughly mixed with mRNA encoding the novel coronavirus spike protein receptor binding domain (S-RBD). 18-24 hours after transfection, the cell culture supernatant was collected, and the expression level of RBD protein was analyzed using an ELISA kit (Sino Biological).

Epo: human non-small cell lung cancer cells (A549) were purchased from ATCC. 10% heat-inactivated fetal bovine serum (Gibco-FBS) (Life Technologies, Thermofisher) was added to F-12K medium (ATCC, 30-2004). Cells were seeded at 35,000-40,000 cells per well in a 6-well flat-bottom cell culture plate (Corning) and transfected after 24 hours. Cells were transfected with mRNA using Lipofectamine 2000 (Thermo Fisher Scientific). The cell culture supernatant was collected 18-24 hours after transfection, and the expression level of EPO protein in the cell supernatant was analyzed using an ELISA kit (R&D systems).

Rabies: human non-small cell lung cancer cells (A549) were purchased from ATCC. 10% heat-inactivated fetal bovine serum (Gibco-FBS) (Life Technologies, Thermofisher) was added to F-12K medium (ATCC, 30-2004). Cells were seeded at 35,000-40,000 cells per well in a 6-well flat-bottom cell culture plate (Corning) and transfected after 24 hours. Cells were transfected with mRNA using Lipofectamine 2000 (Thermo Fisher Scientific). Cells were collected 18-24 hours after transfection and analyzed by flow cytometry. The transfected cells were collected and added to 96-well plates, centrifuged, and then blocked with 2% BSA. After blocking, the cells were incubated with pre-diluted mouse IgG1 antibody against rabies virus glycoprotein G at 4°C for 1 hour. Following incubation, R-phycoerythrin AffiniPure^{™} goat antimouse IgG Fcγ fragment was added and incubated for 30 min. After washing, the cells were resuspended in DPBS and subjected to flow cytometry analysis.

### 1.11 Hematological and blood biochemical analysis

Hematology analysis was performed using an automated hematology analyzer (Sysmex xs-800i) with its matched reagents to measure blood samples.

| **Detection item** | **English abbreviations** | **Unit** | **Detection method** |
|---|---|---|---|
| White blood cell count | WBC | ×10³/µL | Laser scattering + light absorption + peroxidase reaction |
| Neutrophils | NEUT | ×10³/µL&% | Laser scattering + light absorption + peroxidase reaction |
| Lymphocytes | LYMP | ×10³/µL&% | Laser scattering + light absorption + peroxidase reaction |
| Monocytes | MONO | ×10³/µL&% | Laser scattering + light absorption + peroxidase reaction |
| Eosinophils | EOS | ×10³/µL&% | Laser scattering + light absorption + peroxidase reaction |
| Platelet count | PLT | ×10³/µL | Laser scattering method |

Blood biochemical samples were tested using an automatic biochemical analyzer (Hitachi 7060).

| **Detection item** | **English abbreviations** | **Unit** | **Detection method** |
|---|---|---|---|
| Alanine aminotransferase | ALT | U/L | Alanine substrate method |
| Aspartate aminotransferase | AST | U/L | Aspartic acid substrate method |
| Alkaline phosphatase | ALP | U/L | NPP substrate-AMP buffer method |
| Lactate dehydrogenase | LDH | U/L | Lactic acid substrate rate method |
| Urea | UREA | mmol/L | Ureic acid glutamate dehydrogenase method |
| Creatinine | CRE | µmol/L | Sarcosine oxidase method |

### Example 2: Comparison of LiCl method and magnetic bead method

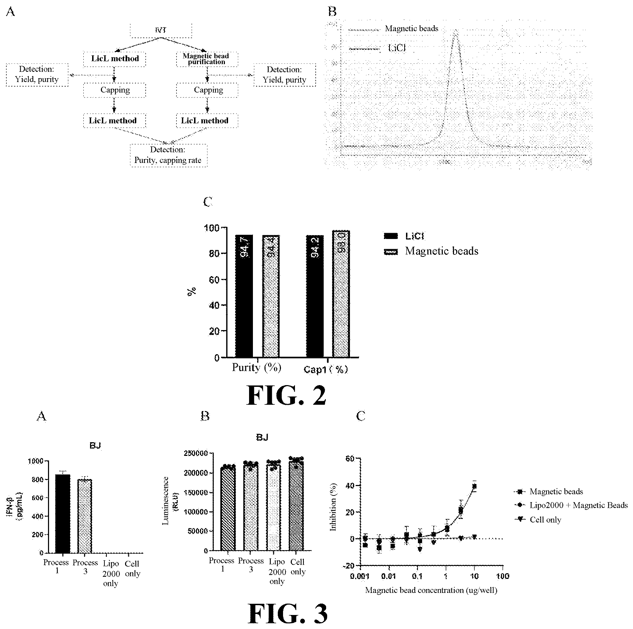

Using sample 1, the experimental protocol shown in panel A in FIG. 2 was used to perform the LiCl method and the magnetic bead method, respectively. The purity test results of the IVT reaction solution purified by LiCl and magnetic beads before capping are shown in panel B in FIG. 2. The purity for the two methods was basically the same, and the yield for magnetic bead purification was 88.4% of LiCl.

The IVT samples purified by using LiCl and magnetic bead methods were capped separately (of the same magnitude), and the capping purity was basically the same, but the capping rate of magnetic bead purification was higher than that of LiCl method, as shown in panel C in FIG. 2. This showed that the magnetic bead method had certain advantages over the LiCl method.

### Example 3: Analysis of impurities introduced by magnetic bead method

Sample 2 was subjected to positive-pressure affinity chromatography followed by magnetic bead method buffer exchange and concentration. IVT, LiCl purification, capping and positive-pressure affinity chromatography were performed. After affinity chromatography, buffer exchange and concentration were performed by using the magnetic bead method, with additional bottom aspiration to remove residual magnetic beads. Then, a 0.22 µm membrane was used for filtration to remove bacteria and also to remove magnetic beads for a second time (Process 3). Processes 1 and 2 were also performed for comparison. Fe element was detected by ICP-MS to evaluate the residue of magnetic beads, and cell viability (see 1.10.3 Cell viability detection) and in vitro innate immune assay (see 1.10.1 In vitro IFN-β detection) were performed, and cytotoxicity of magnetic beads was performed in BC2C12 mouse muscle cells (see 1.10.4 Cytotoxicity detection). The specific test results are shown in Table 1 and FIG. 3.
Process 1: the same as Process 3, except that ultrafiltration buffer exchange and concentration were used instead of magnetic bead method buffer exchange and concentration
Process 2: 0.02 mg of magnetic beads were added to the sample obtained in Process 1 (magnetic bead concentration 0.01 mg/mL)
Process 3: positive-pressure affinity chromatography followed by magnetic bead method buffer exchange and concentration

**Table 1: Fe element detected by ICP-MS**

| Process | Fe (ppm) | Treatment method (all magnetic beads were 10 mg/ml) |
|---|---|---|
| 1 | 0 | Ultrafiltration tube buffer exchange and concentration |
| 2 | 0.99 | 0.02 mg of magnetic beads were added to the sample obtained in Process 1 |
| 3 | 0.26 | Magnetic bead buffer exchange and concentration (bottom aspiration + 0.22 µm filtration) |

As shown in panels A and B in FIG. 3, compared with Process 1, Process 3 exhibited slightly lower in vitro IFN-β detection results but slightly higher cell viability assay results. According to the results of Fe element (Table 1), after positive-pressure affinity chromatography, magnetic bead method buffer exchange and concentration were performed, and the obtained residual magnetic beads were less than 0.01 mg/ml. Combined with cytotoxicity assay of magnetic beads in BC2C12 mouse muscle cells (see panel C in FIG. 3), it indicated that the current residue amount of magnetic beads was essentially non-toxic to cells.

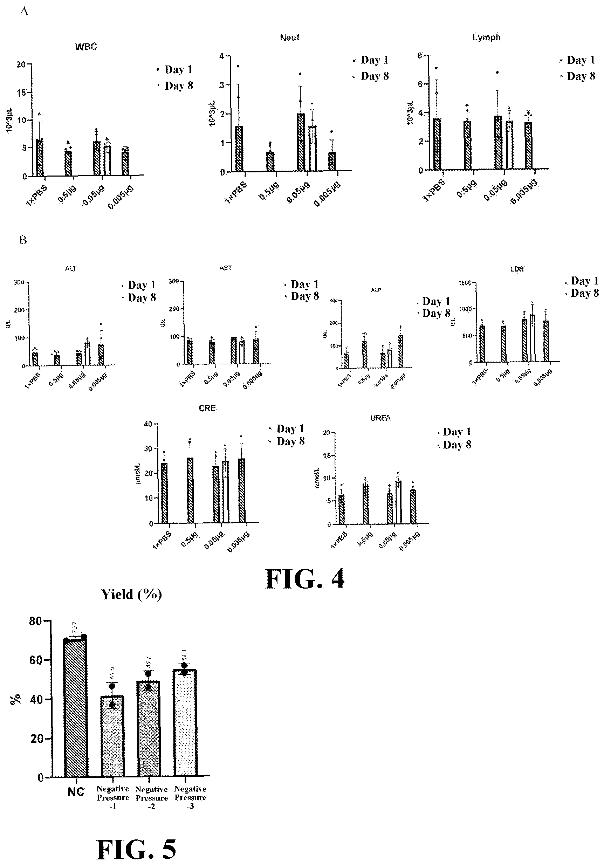

Meanwhile, to investigate whether the introduced impurities would have any impact in mice, a certain amount of magnetic beads (200 µL) was injected into the mice via tail vein. Blood was collected one day later, and a group of mice (0.05 µg) was retained until day 8 for secondary blood collection to assess hematology and blood biochemistry. The results are shown in panels A and B of FIG. 4.

The results showed low toxicity to animals even at the highest dose of 0.5 µg. On day 8 (0.05 µg), it was found that all indicators rebounded.

### Example 4: Negative-pressure affinity chromatography

Affinity chromatography was performed under negative pressure. It was found that the chromatographic packing was deformed due to the instantaneous force of negative pressure, and the yield was lower than that of positive-pressure affinity chromatography, as shown in FIG. 5.

### Example 5: Comparative experiment 1

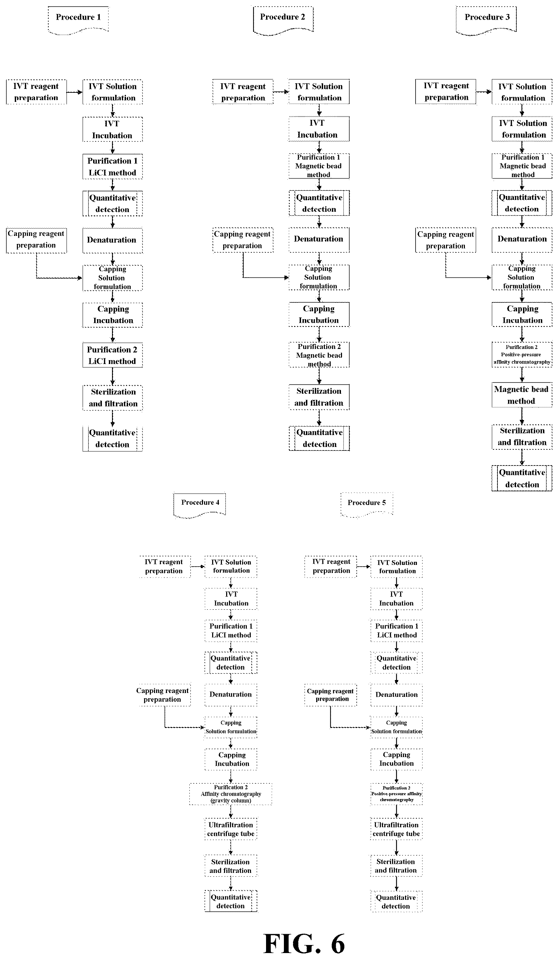

Samples 3, 4, 1, and 5 were selected to prepare two types of linearized plasmids with/without Poly(A). The linearized plasmids were mixed at an 8:2 ratio for IVT. The LiCl method and magnetic bead method were chosen for purification 1. The obtained uncapped products were then capped and subjected to purification 2 using the LiCl method, magnetic bead method, and positive-pressure affinity chromatography plus magnetic bead method, respectively. The specific procedures followed those outlined in Procedures 1-3 of FIG. 6. The resulting samples were analyzed for mRNA purity (referred to as FA purity) using the Agilent Fragment Analyzer, along with capping rate and protein residue tests and other tests. Meanwhile, samples 3 and 4 were selected for in vitro expression in HEK293T cells, while samples 3, 4, 1, and 5 were tested for in vitro expression in A549 cells. The results are as follows:

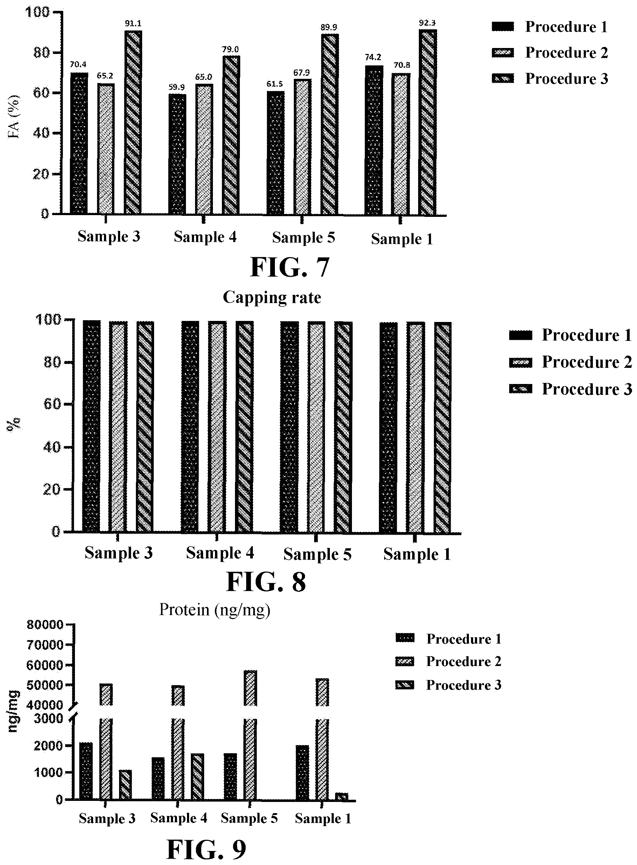

the FA purity for Procedure 3 was higher than those for Procedure 1 and Procedure 2, as shown in FIG. 7; the capping rate showed minimal differences, all exceeding 99%, as shown in FIG. 8; the protein detection results for Procedure 2 was significantly higher than those for Procedure 1 and Procedure 3, and Procedure 3 was the lowest, as shown in FIG. 9;

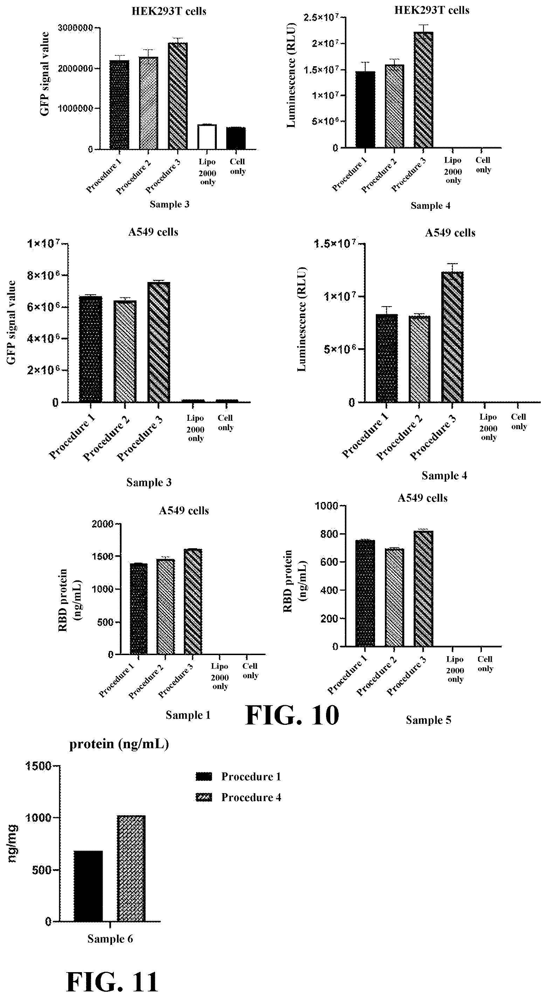

the in vitro expression results for Procedure 3 was higher than those for Procedure 1 and Procedure 2, as shown in FIG. 10.

Sample 6 was used for mRNA preparation. After IVT, LiCl method was selected for purification 1. The obtained uncapped product was capped, followed LiCl method and affinity chromatography for purification 2. The affinity chromatography employed a gravity column, where samples and buffers were allowed to drip naturally under gravity without additional pressure. After affinity chromatography, buffer exchange was performed using an ultrafiltration tube, following the specific procedures outlined in Procedure 1 and Procedure 4 of FIG. 6. The obtained sample was tested for protein residue, and it was found that compared with Procedure 1, Procedure 4 had a poor protein removal effect, as shown in FIG. 11.

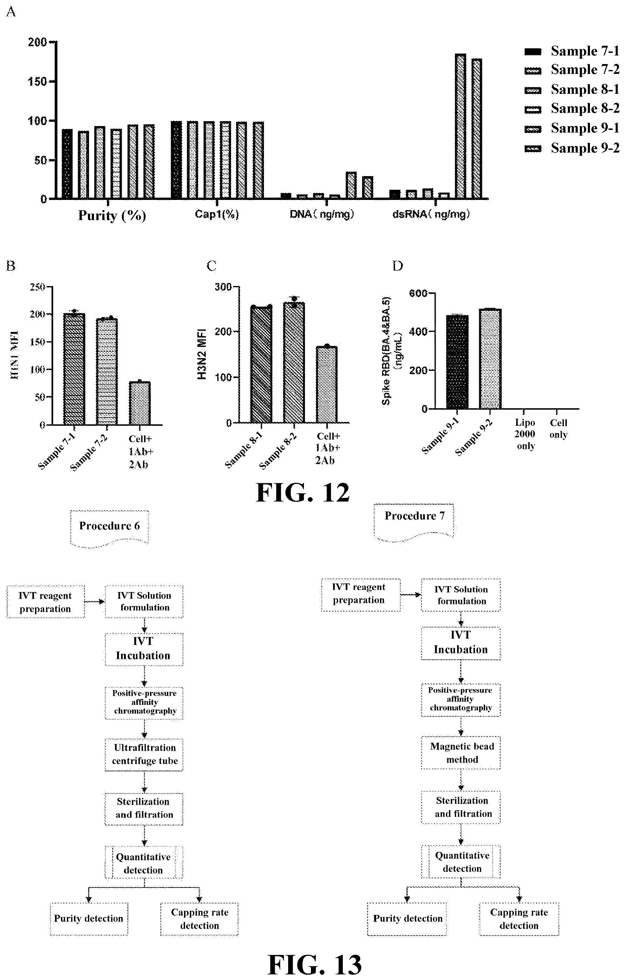

The comparative experiments of Procedures 3 and 5 were performed using samples 7, 8, and 9, in which samples 7-1, 8-1, and 9-1 were subjected to mRNA sample preparation according to Procedure 5, and samples 7-2, 8-2, and 9-2 were subjected to mRNA sample preparation according to Procedure 3 (automated procedure). The obtained mRNA stock solution was subjected to relevant tests (including purity, capping rate, DNA residue, dsRNA residue), innate immunity (INF-β) test, and in vitro protein expression test, with the results shown in FIG. 12 and Table 2.

**Table 2: Physicochemical properties (purity, capping rate, DNA residue, dsRNA residue) and innate immunity (INF-β) results of three samples**

| Name | Purity (%) | Cap1 (%) | DNA (ng/mg) | dsRNA (ng/mg) | IFN-β (pg/mL) |
|---|---|---|---|---|---|
| Sample 7-1 | 89.4 | 99.7 | 7.98 | 12 | < 39.065 |
| Sample 7-2 | 87 | 99.7 | 6.18 | 12 | < 39.065 |
| Sample 8-1 | 93.1 | 99.4 | 7.84 | 14 | < 39.065 |
| Sample 8-2 | 89.8 | 99.4 | 5.87 | 9 | < 39.065 |
| Sample 9-1 | 95.2 | 98.7 | 35.16 | 185 | < 39.065 |
| Sample 9-2 | 95.6 | 98.7 | 29.48 | 179 | < 39.065 |

The test results showed that the detection results of mRNA purity, capping rate, DNA residue and dsRNA residue were basically the same without significant difference, and the DNA residue and dsRNA residue in Procedure 3 were relatively lower, as shown in panel A in FIG. 12. The innate immunity (INF-β) was all below the detection line, as shown in Table 2. The in vitro expression levels of the three samples did not differ significantly, as shown in panels B, C and D in FIG. 12.

### Example 6: Comparative experiment 2

11 samples were selected to prepare mRNA of Cap 1 according to Procedure 6 and Procedure 7 in FIG. 13, respectively, in which m7GpppAmpU was added to samples 10 to 17, m7GpppAmpG was added to samples 18 to 20, and samples were prepared by co-transcription. The prepared samples were subjected to physicochemical property tests including FA purity, capping rate, physicochemical properties (DNA residue, dsRNA residue, protein residue).

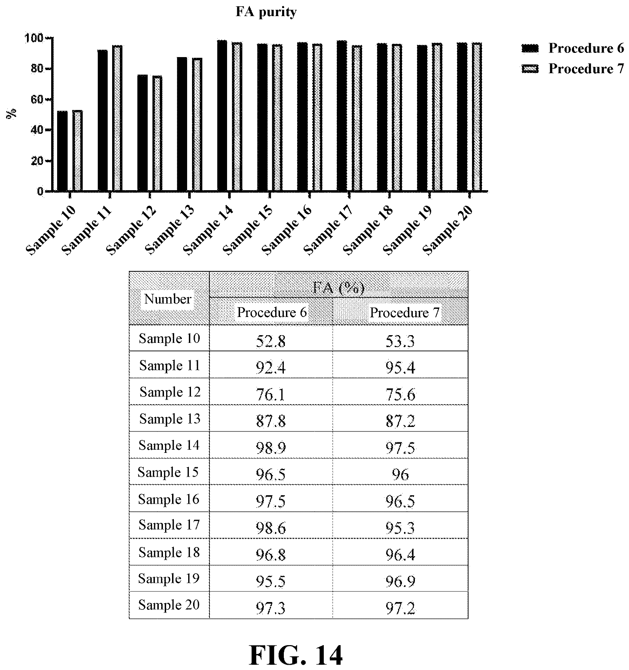

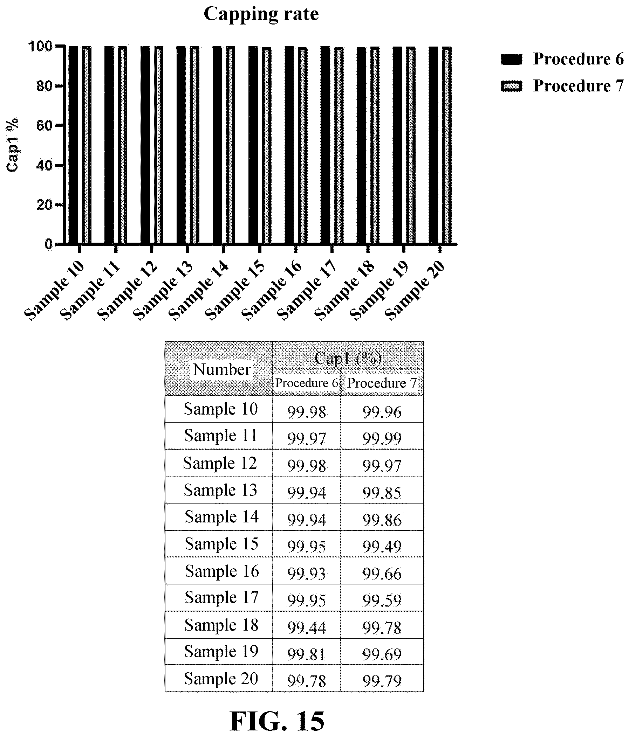

The FA and capping rate of 11 samples were basically the same. See FIG. 14 and FIG. 15 for specific data.

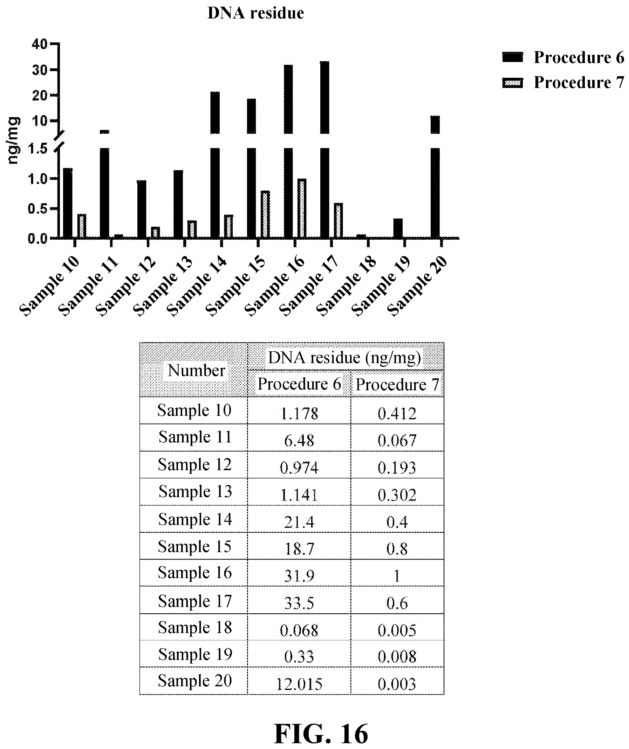

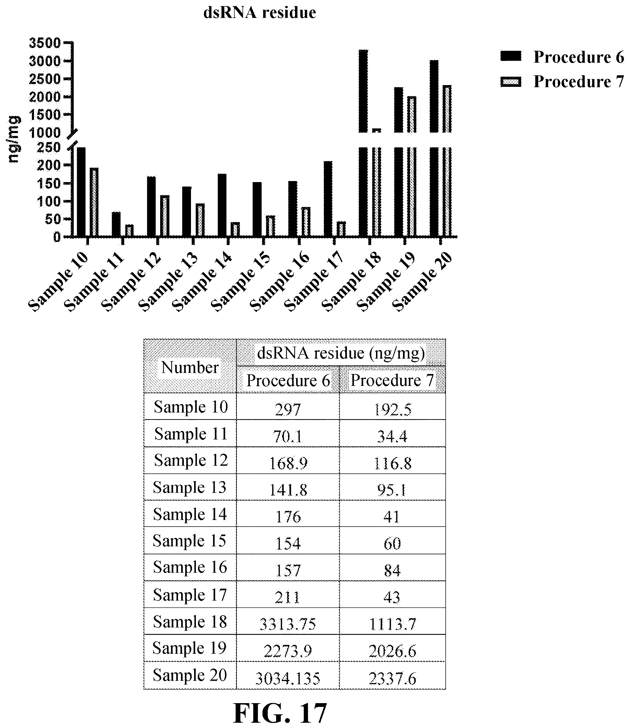

The detection of physicochemical properties showed that the protein residue in the two procedures was not detected. For the DNA residue, Procedure 7 was significantly lower than Procedure 6, and the specific data are shown in FIG. 16. For the dsRNA residue, Procedure 7 was significantly lower than Procedure 6, and the specific data are shown in FIG. 17.

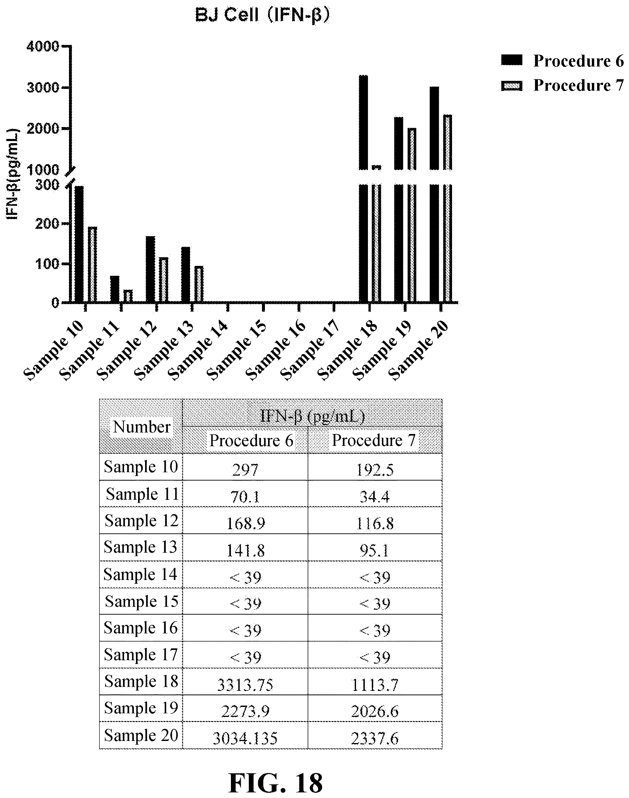

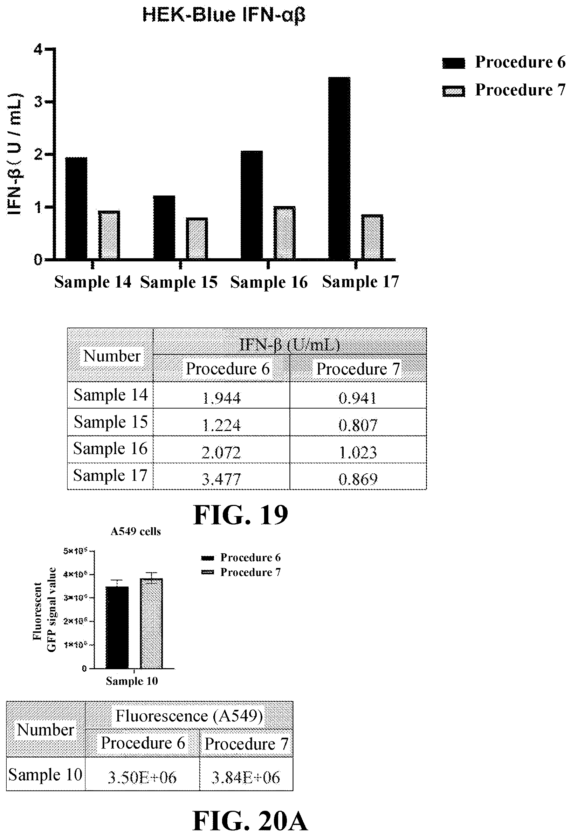

The samples were tested for immunologic factor IFN-β using BJ fibroblasts, and the results showed that the stimulation of IFN-β in Procedure 7 was lower than that in Procedure 6, see FIG. 18, in which samples 14, 15, 16 and 17 were all lower than the detection value in this method. These 4 samples were tested by another method (IFN-α/β Reporter HEK 293 cell assay to detect the content of IFN-β in supernatant from transfected BJ cells), and the results showed that the stimulation of IFN-β in Procedure 7 was lower than that in Procedure 6, the specific data are shown FIG. 19.

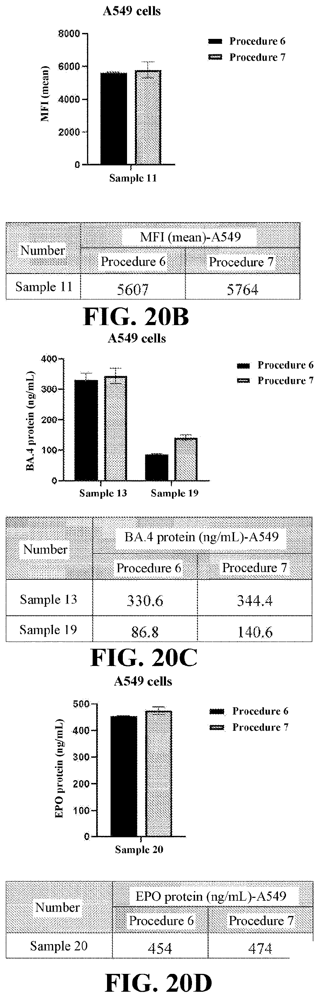

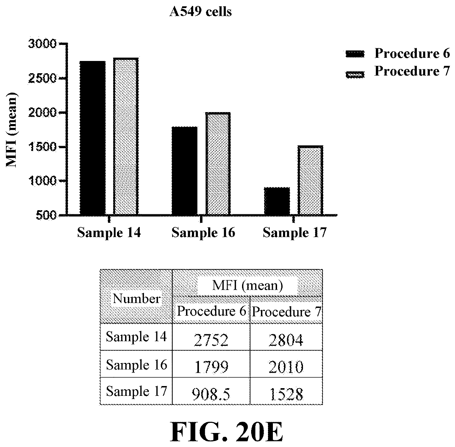

The samples were tested for protein expression in vitro in A549 cells, and the results showed that the differences varied among each sample, but overall, the protein expression level of Procedure 7 was slightly higher than that of Procedure 6, see FIGs. 20A-20E.

Although the present invention has been described with reference to specific examples, those skilled in the art will appreciate that many modifications and variations can be made to the present invention. Therefore, it is understood that the appended claims are intended to cover all such modifications and variations as fall within the true spirit and scope of the present invention.

### SEQUENCE INFORMATION:

Sample 1 (encoding protein: novel coronavirus delta variant strain RBD): SEQ ID NO: 1 
GGGGGGAUCCAAGCUUAGCUUGCUUGUUCUUUUUGCAGAAGCUCAGAAUAA
ACGCUCAACUUUGGCGCCGCCACCAUGGACUGGACCUGGAUUCUCUUCUUGGUG
GCAGCAGCCACGCGAGUCCACUCCAGAGUGCAGCCUACCGAGAGCAUCGUGAGA
UUCCCUAAUAUCACCAAUCUGUGCCCUUUCGGCGAGGUGUUCAAUGCCACCAGA
UUCGCCAGCGUGUACGCAUGGAACCGCAAGCGGAUAAGCAAUUGCGUGGCCGAC
UACAGCGUGCUGUACAAUAGCGCCAGCUUCAGCACCUUCAAAUGUUAUGGUGUU
UCGCCAACAAAGCUGAAUGACCUGUGCUUCACCAAUGUGUACGCCGACAGCUUC
GUGAUCAGAGGCGACGAGGUGAGACAGAUCGCGCCAGGGCAGACCGGCAAGAUC
GCCGACUACAAUUACAAGCUGCCUGACGACUUCACCGGCUGCGUGAUCGCGUGG
AACUCUAACAAUCUAGAUUCGAAAGUUGGAGGCAAUUACAAUUACCGGUACAGA
CUGUUCAGAAAGAGCAAUCUGAAGCCUUUCGAGAGAGACAUCAGCACCGAGAUC
UACCAGGCCGGCAGCAAACCGUGUAAUGGCGUGGAGGGCUUCAAUUGCUACUUC
CCUCUGCAGAGCUACGGCUUCCAGCCUACCAAUGGCGUGGGCUACCAGCCUUAC
AGAGUGGUGGUGCUGAGCUUCGAGCUGCUGCACGCUCCCGCUACCGUGUGCGGC
CCUAAGAAGAGCACCAAUCUGGUGAAGAAUAAGUGCGUGAAUUUCUGAUAAGA
AUUCUAGGCUGGAGCCUCGGUGGCCAUGCUUCUUGCCCCUUGGGCCUCCCCCCA
GCCCCUCCUCCCCUUCCUGCACCCGUACCCCCGUGGUCUUUGAAUAAAGUCUGAG
 
UGGGCGGCACUAGUAAAAAAAAAAAAAAAAAAAAAAAAAAAAAAAAAAAA
AAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
A
Sample 2 (encoding protein Epo): SEQ ID NO: 2 
GGGGGGAUCCGCAGGAGCCAGGGCUGGGCAUAAAAGUCAGGGCAGAGCCAUC
UAUUGCUUACAUUUGCUUCUGACACAACUGUGUUCACUAGCAACCUCAAACAGA
CACCAUGGGGGUGCACGAAUGUCCUGCCUGGCUGUGGCUUCUCCUGUCCCUGCU
GUCGCUCCCUCUGGGCCUCCCAGUCCUGGGCGCCCCACCACGCCUCAUCUGUGAC
AGCCGAGUCCUGGAGAGGUACCUCUUGGAGGCCAAGGAGGCCGAGAAUAUCACG
ACGGGCUGUGCUGAACACUGCAGCUUGAAUGAGAAUAUCACUGUCCCAGACACC
AAAGUUAAUUUCUAUGCCUGGAAGAGGAUGGAGGUCGGGCAGCAGGCCGUAGA
AGUCUGGCAGGGCCUGGCCCUGCUGUCGGAAGCUGUCCUGCGGGGCCAGGCCCU
GUUGGUCAACUCUUCCCAGCCGUGGGAGCCCCUGCAGCUGCAUGUGGAUAAAGC
CGUCAGUGGCCUUCGCAGCCUCACCACUCUGCUUCGGGCUCUGGGAGCCCAGAA
GGAAGCCAUCUCCCCUCCAGAUGCGGCCUCAGCUGCUCCACUCCGAACAAUCACU
GCUGACACUUUCCGCAAACUCUUCCGAGUCUACUCCAAUUUCCUCCGGGGAAAG
CUGAAGCUGUACACAGGGGAGGCCUGCAGGACAGGGGACAGAUGAUAAGAAUUC
GCUCGCUUUCUUGCUGUCCAAUUUCUAUUAAAGGUUCCUUUGUUCCCUAAGUCC
AACUACUAAACUGGGGGAUAUUAUGAAGGGCCUUGAGCAUCUGGAUUCUGCCUA
AUAAAAAACAUUUAUUUUCAUUGCACUAGUAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAA
Sample 3 (encoding protein: eGFP): SEQ ID NO: 3 
GGGGGGAUCCAAGCUUAGCUUGCUUGUUCUUUUUGCAGAAGCUCAGAAUAA
ACGCUCAACUUUGGCGCCGCCACCAUGGUGAGCAAGGGCGAGGAGCUGUUCACC
GGGGUGGUGCCCAUCCUGGUCGAGCUGGACGGCGACGUAAACGGCCACAAGUUC
AGCGUGUCCGGCGAGGGCGAGGGCGAUGCCACCUACGGCAAGCUGACCCUGAAG
UUCAUCUGCACCACCGGCAAGCUGCCCGUGCCCUGGCCCACCCUCGUGACCACCC
UGACCUACGGCGUGCAGUGCUUCAGCCGCUACCCCGACCACAUGAAGCAGCACG
ACUUCUUCAAGUCCGCCAUGCCCGAAGGCUACGUCCAGGAGCGCACCAUCUUCU
UCAAGGACGACGGCAACUACAAGACCCGCGCCGAGGUGAAGUUCGAGGGCGACA
CCCUGGUGAACCGCAUCGAGCUGAAGGGCAUCGACUUCAAGGAGGACGGCAACA
UCCUGGGGCACAAGCUGGAGUACAACUACAACAGCCACAACGUCUAUAUCAUGG
 
CCGACAAGCAGAAGAACGGCAUCAAGGUGAACUUCAAGAUCCGCCACAACAUCG
AGGACGGCAGCGUGCAGCUCGCCGACCACUACCAGCAGAACACCCCCAUCGGCGA
CGGCCCCGUGCUGCUGCCCGACAACCACUACCUGAGCACCCAGUCCGCCCUGAGC
AAAGACCCCAACGAGAAGCGCGAUCACAUGGUCCUGCUGGAGUUCGUGACCGCC
GCCGGGAUCACUCUCGGCAUGGACGAGCUGUACAAGUGAUAAGAAUUCUAGGCU
GGAGCCUCGGUGGCCAUGCUUCUUGCCCCUUGGGCCUCCCCCCAGCCCCUCCUCC
CCUUCCUGCACCCGUACCCCCGUGGUCUUUGAAUAAAGUCUGAGUGGGCGGCAC
UAGUAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAA
Sample 4 (encoding protein: Luc): SEQ ID NO: 4 
GGGGGGAUCCAAGCUUAGCUUGCUUGUUCUUUUUGCAGAAGCUCAGAAUAA
ACGCUCAACUUUGGCGCCGCCACCAUGGAAGAUGCGAAGAACAUCAAGAAGGGA
CCUGCCCCGUUUUACCCUUUGGAGGACGGUACAGCAGGAGAACAGCUCCACAAG
GCGAUGAAACGCUACGCCCUGGUCCCCGGAACGAUUGCGUUUACCGAUGCACAU
AUUGAGGUAGACAUCACAUACGCAGAAUACUUCGAAAUGUCGGUGAGGCUGGCG
GAAGCGAUGAAGAGAUAUGGUCUUAACACUAAUCACCGCAUCGUGGUGUGUUCG
GAGAACUCAUUGCAGUUUUUCAUGCCGGUCCUUGGAGCACUUUUCAUCGGGGUC
GCAGUCGCGCCAGCGAACGACAUCUACAAUGAGCGGGAACUCUUGAAUAGCAUG
GGAAUCUCCCAGCCGACGGUCGUGUUUGUCUCCAAAAAGGGGCUGCAGAAAAUC
CUCAACGUGCAGAAGAAGCUCCCCAUUAUUCAAAAGAUCAUCAUUAUGGAUAGC
AAGACAGAUUACCAAGGGUUCCAGUCGAUGUAUACCUUUGUGACAUCGCAUUUG
CCGCCAGGGUUUAACGAGUAUGACUUCGUCCCCGAGUCAUUUGACAGAGAUAAA
ACCAUCGCGCUGAUUAUGAACUCCUCGGGUAGCACCGGUUUGCCAAAGGGGGUG
GCGUUGCCCCACCGCACUGCUUGUGUGCGGUUCUCGCACGCUAGGGACCCUAUC
UUUGGUAAUCAGAUCAUUCCCGACACAGCAAUCCUGUCCGUGGUACCUUUUCAU
CACGGUUUUGGCAUGUUCACGACUCUCGGCUAUUUGAUUUGCGGUUUCAGGGUC
GUACUUAUGUAUCGGUUCGAGGAAGAACUGUUUUUGAGAUCCUUGCAAGAUUA
CAAGAUCCAGUCGGCCCUCCUUGUGCCAACGCUUUUCUCAUUCUUUGCGAAAUC
GACACUUAUUGAUAAGUAUGACCUUUCCAAUCUGCAUGAGAUUGCCUCAGGGGG
ΛGCGCCGCUUΛGCΛΛGGΛΛGUCGGGGΛGGCΛGUGGCCΛΛGCGCUUCCΛCCUUCC
CGGAAUCCGGCAGGGAUACGGGCUCACGGAGACAACAUCCGCGAUCCUUAUCAC
GCCCGAGGGUGACGAUAAGCCGGGAGCCGUCGGAAAAGUGGUCCCCUUCUUUGA
AGCCAAGGUCGUAGACCUCGACACGGGAAAAACCCUCGGAGUGAACCAGAGGGG
CGAGCUCUGCGUGAGAGGGCCGAUGAUCAUGUCAGGUUACGUGAAUAACCCUGA
 
AGCGACGAAUGCGCUGAUCGACAAGGAUGGGUGGUUGCAUUCGGGAGACAUUGC
CUAUUGGGAUGAGGAUGAGCACUUCUUUAUCGUAGAUCGACUUAAGAGCUUGA
UCAAAUACAAAGGCUAUCAGGUAGCGCCUGCCGAGCUCGAGUCAAUCCUGCUCC
AGCACCCCAACAUUUUCGACGCCGGAGUGGCCGGGUUGCCCGAUGACGACGCGG
GUGAGCUGCCAGCGGCCGUGGUAGUCCUCGAACAUGGGAAAACAAUGACCGAAA
AGGAGAUCGUGGACUACGUAGCAUCACAAGUGACGACUGCGAAGAAACUGAGGG
GAGGGGUAGUCUUUGUGGACGAGGUCCCGAAAGGCUUGACUGGGAAGCUUGACG
CUCGCAAAAUCCGGGAAAUCCUGAUUAAGGCAAAGAAAGGCGGGAAAAUCGCUG
UCUGAUAAUAGGCUGGAGCCUCGGUGGCCAUGCUUCUUGCCCCUUGGGCCUCCC
CCCAGCCCCUCCUCCCCUUCCUGCACCCGUACCCCCGUGGUCUUUGAAUAAAGUC
UGAGUGGGCGGCACUAGUAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAA
Sample 5 (encoding protein: novel coronavirus RBD): SEQ ID NO: 5 
GGGGGGAUCCAAGCUUAGCUUGCUUGUUCUUUUUGCAGAAGCUCAGAAUAA
ACGCUCAACUUUGGCGCCGCCACCAUGGACUGGACCUGGAUUCUCUUCUUGGUG
GCAGCAGCCACGCGAGUCCACUCCAGAGUCCAACCAACAGAAUCUAUUGUUAGA
UUUCCUAAUAUUACAAACUUGUGCCCUUUUGGUGAAGUUUUUAACGCCACCAGA
UUUGCAUCUGUUUAUGCUUGGAACAGGAAGAGAAUCAGCAACUGUGUUGCUGA
UUAUUCUGUCCUAUAUAAUUCCGCAUCAUUUUCCACUUUUAAGUGUUAUGGAGU
GUCUCCUACUAAAUUAAAUGAUCUCUGCUUUACUAAUGUCUAUGCAGAUUCAUU
UGUAAUUAGAGGUGAUGAAGUCAGACAAAUCGCUCCAGGGCAAACUGGAAAGA
UUGCUGAUUAUAAUUAUAAAUUACCAGAUGAUUUUACAGGCUGCGUUAUAGCU
UGGAACUCUAACAAUCUUGAUUCUAAGGUUGGUGGUAAUUAUAAUUACCUGUA
UAGAUUGUUUAGGAAGUCUAAUCUCAAACCUUUUGAGAGAGAUAUUUCAACUG
AAAUCUAUCAGGCCGGUAGCACACCUUGUAAUGGUGUUGAAGGUUUUAAUUGU
UACUUUCCUUUACAAUCAUAUGGUUUCCAACCCACUAAUGGUGUUGGUUACCAA
CCAUACAGAGUAGUAGUACUUUCUUUUGAACUUCUACAUGCACCAGCAACUGUU
UGUGGACCUAAAAAGUCUACUAAUUUGGUUAAAAACAAAUGUGUCAAUUUCUG
AUAAGAAUUCUAGGCUGGAGCCUCGGUGGCCAUGCUUCUUGCCCCUUGGGCCUC
CCCCCAGCCCCUCCUCCCCUUCCUGCACCCGUACCCCCGUGGUCUUUGAAUAAAG
UCUGAGUGGGCGGCACUAGUAAAAAAAAAAAAAAAAAAAAAAAAAAAAAAAA
AAAAAAAAAAAAAAAAAAAAAAAAAAAAAAAAAAAAAAAAAAAAAAAAAAAA
 
AAAAAAAAAAAAAAAAAAAAAAAAAAAAAAAAAAAAAAAAAAAAAAAAAAAAA

AAAAAAA
Sample 6 (encoding protein: murine IL-12): SEQ ID NO: 6 
GGGGGGAUCCAAGCUUAGCUUGCUUGUUCUUUUUGCAGAAGCUCAGAAUAA
ACGCUCAACUUUGGCGCCGCCACCAUGUGUCCUCAGAAGCUAACCAUCUCCUGG
UUUGCCAUCGUUUUGCUGGUGUCUCCACUCAUGGCCAUGUGGGAGCUGGAGAAA
GACGUUUAUGUUGUAGAGGUGGACUGGACUCCCGAUGCCCCUGGAGAAACAGUG
AACCUCACCUGUGACACGCCUGAAGAAGAUGACAUCACCUGGACCUCAGACCAG
AGACAUGGAGUCAUAGGCUCUGGAAAGACCCUGACCAUCACUGUCAAAGAGUUC
CUAGAUGCUGGCCAGUACACCUGCCACAAAGGAGGCGAGACUCUGAGCCACUCA
CAUCUGCUGCUCCACAAGAAGGAAAAUGGAAUUUGGUCCACUGAAAUUUUAAAA
AAUUUCAAAAACAAGACUUUCCUGAAGUGUGAAGCACCAAAUUACUCCGGACGG
UUCACGUGCUCAUGGCUGGUGCAAAGAAACAUGGACUUGAAGUUCAACAUCAAG
AGCAGUAGCAGUUCCCCUGACUCUCGGGCAGUGACAUGUGGAAUGGCGUCUCUG
UCUGCAGAGAAGGUCACACUGGACCAAAGGGACUAUGAGAAGUAUUCAGUGUCC
UGCCAGGAGGAUGUCACCUGCCCAACUGCCGAGGAGACACUGCCCAUUGAACUG
GCGUUGGAAGCACGGCAGCAGAAUAAAUAUGAGAACUACAGCACCAGCUUCUUC
AUCAGGGACAUCAUCAAACCAGACCCGCCCAAGAACUUGCAGAUGAAGCCUUUG
AAGAACUCACAGGUGGAGGUCAGCUGGGAGUACCCUGACUCCUGGAGCACUCCC
CAUUCCUACUUCUCCCUCAAGUUCUUUGUUCGAAUCCAGCGCAAGAAAGAAAAG
AUGAGGAGACAGAGGAGGGGUGUAACCAGAAAGGUGCGUUCCUCGUAGAGAAG
ACAUCUACCGAAGUCCAAUGCAAAGGCGGGAAUGUCUGCGUGCAAGCUCAGGAU
CGCUAUUACAAUUCCUCAUGCAGCAAGUGGGCAUGUGUUCCCUGCAGGGUCCGA
UCCGGAGGCGGGGGAGCGGAGGCGGAGGGAGCGGAGGCGGAGGGAGCAGGGUCA
UUCCAGUCUCUGGACCUGCCAGGUGUCUUAGCCAGUCCCGAAACCUGCUGAAGA
CCACAGAUGACAUGGUGAAGACGGCCAGAGAAAAACUGAAACAUUAUUCCUGCA
CUGCUGAAGACAUCGAUCAUGAAGACAUCACACGGGACCAAACCAGCACAUUGA
AGACCUGUUUACCACUGGAACUACACAAGAACGAGAGUUGCCUGGCUACUAGAG
AGACUUCUUCCACAACAAGAGGGAGCUGCCUGCCCCCACAGAAGACGUCUUUGA
UGAUGACCCUGUGCCUUGGUAGCAUCUAUGAGGACUUGAAGAUGUACCAGACAG
AGUUCCAGGCCAUCAACGCAGCACUUCAGAAUCACAACCAUCAGCAGAUCAUUU
UAGACAAGGGCAUGCUGGUGGCCAUCGAUGAGCUGAUGCAGUCUCUGAAUCAUA
AUGGCGAGACUCUGCGCCAGAAACCUCCUGUGGGAGAAGCAGACCCUUACAGAG
UGAAAAUGAAGCUCUGCAUCCUGCUUCACGCCUUCAGCACCCGCGUCGUGACCA
UCAACAGGGUGAUGGGCUAUCUGAGCUCCGCCUGAUAAUAGGCUGGAGCCUCGG
 
UGGCCAUGCUUCUUGCCCCUUGGGCCUCCCCCCAGCCCCUCCUCCCCUUCCUGCA
CCCGUACCCCCGUGGUCUUUGAAUAAAGUCUGAGUGGGCGGCACUAGUAAAAAA
AAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAA
AAAAAAAAAAAAAAAAAAAAAAAAAAAAAAAAAA
Sample 7 (encoding protein: H1N1 HA, U in the sequence employs 100% N1-methyl pseudouridine ( M1 ψ) modification): SEQ ID NO: 7 
GGGGGGAUCCAAGCUUAGCUUGCUUGUUCUUUUUGCAGAAGCUCAGAAUAA
ACGCUCAACUUUGGCGCCGCCACCAUGAAAGCCAUCCUGGUGGUAAUGCUGUAC
ACCUUCACCACAGCCAACGCCGACACCCUGUGCAUCGGCUACCACGCCAACAACA
GCACCGACACCGUGGACACCGUGCUAGAGAAAAACGUGACCGUGACCCACAGCG
UGAACCUGCUGGAGGACAAACACAACGGCAAACUGUGCAAACUGAGAGGCGUGG
CACCCCUGCACCUGGGCAAAUGCAACAUCGCCGGCUGGAUACUGGGAAACCCCG
AGUGCGAGAGCCUGAGCACCGCCAGGAGCUGGAGCUACAUCGUGGAGACAAGCA
ACAGCGACAACGGCACCUGCUACCCAGGCGACUUCAUCAACUACGAGGAGCUGC
GAGAGCAGCUCAGCAGCGUGAGCAGCUUCGAGCGCUUCGAAAUCUUCCCCAAAA
CAAGCAGCUGGCCAAACCACGACAGCGACAACGGCGUGACCGCCGCCUGCCCCCA
CGCCGGCGCCAAAAGUUUCUACAAAAACCUGAUCUGGCUGGUGAAAAAAGGCAA
AAGCUACCCCAAAAUCAACCAGACCUACAUCAACGACAAAGGCAAAGAGGUGCU
CGUGCUGUGGGGCAUCCACCACCCGCCCACCAUCGCAGACCAGCAGAGCCUGUAC
CAGAACGCCGACGCCUACGUGUUCGUGGGCACCAGCCGGUACAGCAAAAAAUUC
AAACCCGAGAUCGCCACCAGACCCAAAGUGAGGGACCAGGAGGGCAGAAUGAAC
UACUACUGGACCCUGGUGGAGCCCGGCGACAAAAUCACCUUCGAGGCCACCGGG
AACCUGGUGGCACCCAGAUACGCCUUCACAAUGGAGAGAGACGCCGGCAGCGGC
AUCAUCAUCAGCGACACCCCCGUGCACGACUGCAACACCACCUGCCAGACCCCCG
AAGGCGCCAUCAACACCAGCCUGCCCUUCCAGAACGUACACCCAAUCACCAUCGG
CAAAUGCCCCAAAUACGUGAAAAGCACCAAACUGAGACUGGCCACCGGCCUGAG
GAACGUGCCCAGCAUCCAGAGCAGGGGCCUGUUCGGCGCCAUCGCCGGCUUCAU
CGAGGGCGGCUGGACCGGCAUGGUGGACGGCUGGUACGGCUACCACCACCAGAA
CGAGCAGGGGAGCGGCUACGCCGCCGACCUGAAAAGCACACAGAACGCCAUCGA
CAAAAUCACCAACAAAGUGAACAGCGUGAUAGAGAAAAUGAACACCCAGUUCAC
CGCCGUGGGCAAAGAGUUCAACCACCUGGAGAAAAGAAUCGAGAACCUGAACAA
AAAAGUGGACGACGGCUUCCUGGACAUCUGGACCUACAACGCCGAGCUGCUGGU
GCUGCUGGAGAACGAGAGAACCCUGGACUACCACGACAGCAACGUGAAAAACCU
GUACGAGAAAGUGCGGAACCAGCUGAAAAACAACGCCAAAGAGAUCGGGAACGG
 
GUGCUUCGAGUUCUACCACAAAUGCGACAACACCUGCAUGGAGAGCGUGAAAAA
CGGCACCUACGACUACCCCAAAUACAGCGAGGAGGCCAAACUGAACAGAGAGAA
AAUCGACGGAGUCAAACUGGACAGCACCAGAAUCUACCAGAUCCUGGCAAUCUA
CAGCACCGUGGCCAGCAGCCUGGUGCUGGUGGUGAGCCUGGGCGCCAUCAGCUU
CUGGAUGUGCAGCAACGGCAGCCUCCAGUGCAGAAUCUGCAUAUGAUAAGAAUU
CUAGGCUGGAGCCUCGGUGGCCAUGCUUCUUGCCCCUUGGGCCUCCCCCCAGCCC
CUCCUCCCCUUCCUGCACCCGUACCCCCGUGGUCUUUGAAUAAAGUCUGAGUGG
GCGGCACUAGUAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAA
Sample 8 (encoding protein: H3N2 HA, U in the sequence employs 100% N1-methyl pseudouridine ( M1 ψ) modification): SEQ ID NO: 8 
GGGGGGAUCCAAGCUUAGCUUGCUUGUUCUUUUUGCAGAAGCUCAGAAUAA
ACGCUCAACUUUGGCGCCGCCACCAUGAAGACCAUCAUCGCUCUGAGCAACAUC
CUGUGCCUGGUGUUCGCCCAGAAGAUCCCUGGCAACGACAACUCCACCGCCACCC
UGUGCCUGGGCCACCACGCCGUGCCCAACGGCACCAUCGUGAAGACCAUCACCAA
CGACAGGAUCGAGGUGACCAAUGCCACCGAGCUGGUGCAGAACUCCUCCAUCGG
GGAGAUCUGCGGCAGCCCCCACCAGAUCCUGGAUGGGGGCAAUUGCACCCUGAU
CGACGCCCUGCUGGGCGAUCCUCAGUGUGAUGGCUUCCAGAAUAAGGAGUGGGA
CCUGUUCGUGGAGAGGAGCAGAGCUAACAGCAACUGCUACCCCUACGACGUGCC
CGACUACGCCUCCCUGAGGAGCCUGGUGGCCAGCAGCGGGACCCUGGAGUUCAA
GAACGAGAGCUUCAAUUGGACCGGCGUGAAGCAGAAUGGCACCAGCUCUGCCUG
CAUCAGAGGGAGCAGCAGCUCCUUCUUCAGCAGGCUGAAUUGGCUGACCAGCCU
GAACAAUAUCUACCCUGCCCAGAACGUGACCAUGCCUAACAAGGAGCAGUUUGA
CAAGCUGUACAUCUGGGGGGUGCACCACCCCGACACCGACAAGAAUCAGAUCUC
UCUGUUCGCCCAGAGCUCCGGCAGGAUCACCGUGAGCACCAAGAGGAGCCAGCA
GGCCGUGAUCCCCAACAUCGGCAGCCGGCCCCGGAUCAGGGACAUCCCUAGCCGG
AUCAGCAUCUACUGGACCAUCGUGAAGCCCGGCGACAUCCUGCUGAUCAAUUCC
ACCGGCAAUCUGAUCGCCCCCAGGGGCUACUUCAAGAUCCGGUCCGGCAAGAGC
AGCAUCAUGAGGAGCGACGCCCCCAUCGGGAAGUGCAAGUCUGAGUGCAUCACC
CCCAACGGGAGCAUCCCCAAUGAUAAGCCCUUCCAGAACGUGAACCGGAUCACU
UACGGGGCCUGCCCACGGUACGUGAAGCAGAGCACCCUGAAGCUGGCCACCGGG
AUGAGGAAUGUGCCCGAGAAGCAGACCAGAGGCAUCUUCGGCGCCAUCGCCGGC
UUCAUCGAGAACGGCUGGGAGGGCAUGGUGGACGGCUGGUACGGCUUCCGGCAC
CAGAACUCUGAGGGCCGGGGCCAGGCCGCCGACCUGAAGAGCACCCAGGCCGCC
 
AUUGAUCAGAUCAAUGGCAAGCUGAACCGGCUGAUCGGGAAGACCAACGAGAAG
UUCCACCAGAUCGAGAAGGAGUUCAGCGAGGUGGAGGGCAGGGUGCAGGACCUG
GAGAAGUACGUGGAGGACACCAAGAUCGACCUGUGGAGCUACAACGCCGAGCUG
CUGGUGGCCCUGGAGAACCAGCACACCAUCGACCUGACCGACAGCGAGAUGAAC
AAGCUGUUCGAGAAGACCAAGAAGCAGCUGCGGGAGAACGCCGAGGACAUGGGC
AACGGGUGCUUCAAGAUCUACCACAAGUGCGACAACGCCUGCAUCGGCAGCAUC
AGGAACGAGACAUACGACCACAACGUGUACAGGGACGAGGCCCUGAACAACAGG
UUCCAGAUCAAGGGCGUGGAGCUGAAGUCCGGCUACAAGGACUGGAUUCUGUGG
AUCAGCUUCGCCAUGAGCUGCUUCCUGCUGUGCAUCGCCCUGCUGGGCUUCAUC
AUGUGGGCCUGCCAGAAGGGCAACAUCAGAUGCAACAUUUGCAUCUGAUAAGAA
UUCUAGGCUGGAGCCUCGGUGGCCAUGCUUCUUGCCCCUUGGGCCUCCCCCCAGC
CCCUCCUCCCCUUCCUGCACCCGUACCCCCGUGGUCUUUGAAUAAAGUCUGAGUG
GGCGGCACUAGUAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAA
Sample 9 (encoding protein: novel coronavirus BA.4/5 RBD, U in the sequence employs 100% N1-Methyl pseudouridine ( M1 ψ) modification): SEQ ID NO: 9 
GGGGGGAUCCAAGCUUAGCUUGCUUGUUCUUUUUGCAGAAGCUCAGAAUAA
ACGCUCAACUUUGGCGCCGCCACCAUGGACUGGACCUGGAUUCUGUUCCUGGUG
GCUGCCGCCACCAGGGUGCACAGCAGGGUGCAGCCCACCGAGAGCAUCGUGAGG
UUCCCCAACAUCACCAACCUGUGCCCCUUCGACGAGGUGUUCAACGCCACCAGGU
UCGCCUCCGUGUACGCCUGGAACCGGAAGAGGAUCAGCAACUGCGUGGCCGACU
ACAGCGUGCUGUACAACUUUGCCCCCUUCUUCGCCUUCAAGUGCUACGGCGUGA
GCCCCACCAAGCUGAACGACCUGUGCUUCACCAACGUGUACGCCGACAGCUUCG
UGAUCAGGGGCAACGAGGUGUCCCAGAUUGCCCCCGGCCAGACCGGGAACAUCG
CUGACUACAACUACAAGCUGCCCGAUGACUUCACCGGCUGCGUGAUCGCCUGGA
ACAGCAACAAGCUGGACAGCAAGGUGGGGGGCAACUACAAUUACCGGUACCGGC
UGUUCCGGAAGAGCAACCUGAAGCCUUUCGAGAGGGACAUCAGCACAGAGAUCU
ACCAGGCCGGCAACAAGCCCUGCAACGGGGUGGCCGGCGUGAACUGUUACUUCC
CCCUGCAGAGCUACGGGUUCAGGCCCACCUACGGGGUGGGCCACCAGCCCUACA
GGGUGGUGGUGCUGAGCUUCGAGCUGCUGCACGCCCCCGCCACCGUGUGUGGGC
CUAAGAAGAGCACCAACCUGGUGAAGAACAAGUGCGUGAACUUCUGAUAAGAAU
UCUAGGCUGGAGCCUCGGUGGCCAUGCUUCUUGCCCCUUGGGCCUCCCCCCAGCC
CCUCCUCCCCUUCCUGCACCCGUACCCCCGUGGUCUUUGAAUAAAGUCUGAGUG
GGCGGCACUAGUAAAAAAAAAAAAAAAAAAAAAAAAAAAAAAAAAAAAAAAA
 
AAAAAAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAAAAAAAA
Sample 10 (encoding protein: eGFP, cap analog: m7GpppAmpU, other U in the sequence employs N1-methylpseudouridine (M1ψ) modification except for the grey marked UTP): SEQ ID NO: 10 
AUGGGCGGCGCAUGAGAGAAGCCCAGACUUGUUCUUUUUGCAGAAGCUCAGA
AUAAACGCUCAACUUUGGCGCCGCCACCAUGGUGAGCAAGGGCGAGGAGCUGUU
CACCGGGGUGGUGCCCAUCCUGGUCGAGCUGGACGGCGACGUAAACGGCCACAA
GUUCAGCGUGUCCGGCGAGGGCGAGGGCGAUGCCACCUACGGCAAGCUGACCCU
GAAGUUCAUCUGCACCACCGGCAAGCUGCCCGUGCCCUGGCCCACCCUCGUGACC
ACCCUGACCUACGGCGUGCAGUGCUUCAGCCGCUACCCCGACCACAUGAAGCAGC
ACGACUUCUUCAAGUCCGCCAUGCCCGAAGGCUACGUCCAGGAGCGCACCAUCU
UCUUCAAGGACGACGGCAACUACAAGACCCGCGCCGAGGUGAAGUUCGAGGGCG
ACACCCUGGUGAACCGCAUCGAGCUGAAGGGCAUCGACUUCAAGGAGGACGGCA
ACAUCCUGGGGCACAAGCUGGAGUACAACUACAACAGCCACAACGUCUAUAUCA
UGGCCGACAAGCAGAAGAACGGCAUCAAGGUGAACUUCAAGAUCCGCCACAACA
UCGAGGACGGCAGCGUGCAGCUCGCCGACCACUACCAGCAGAACACCCCCAUCGG
CGACGGCCCCGUGCUGCUGCCCGACAACCACUACCUGAGCACCCAGUCCGCCCUG
AGCAAAGACCCCAACGAGAAGCGCGAUCACAUGGUCCUGCUGGAGUUCGUGACC
GCCGCCGGGAUCACUCUCGGCAUGGACGAGCUGUACAAGUGAUAAGAAUUCUAG
GCUGGAGCCUCGGUGGCCAUGCUUCUUGCCCCUUGGGCCUCCCCCCAGCCCCUCC
UCCCCUUCCUGCACCCGUACCCCCGUGGUCUUUGAAUAAAGUCUGAGUGGGCGG
CACUAGUAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
Sample 11 (encoding protein: RSV Pre-F, cap analog: m7GpppAmpU, other U in the sequence employs N1-methylpseudouridine (M1ψ) modification except for the grey marked UTP): SEQ ID NO: 11 
AUGGGGAUCCAAGCUUAGCUUGCUUGUUCUUUUUGCAGAAGCUCAGAAUAA
ACGCUCAACUUUGGCGCCGCCACCAUGGAGCUGCUGAUCCUGAAGGCUAAUGCC
AUUACCACCAUUCUGACCGCCGUGACAUUCUGCUUCGCGAGCGGGCAGAACAUC
ACCGAGGAGUUCUACCAGAGCACCUGUUCUGCCGUCAGCAAGGGGUACCUGUCC
GCCCUGCGGACAGGAUGGUAUACCUCAGUGAUCACAAUUGAGCUGUCCAAUAUU
AAGGAGAAUAAGUGCAAUGGGACUGACGCCAAGGUGAAGCUGAUCAAGCAGGA
GCUGGACAAGUACAAGAACGCCGUGACAGAGCUGCAGCUGCUCAUGCAGAGCAC
CCCCGCCACCGGGAGUGGCAGCGCCAUCUGCAGCGGCGUGGCCGUCUGCAAGGU
 
GCUGCACCUCGAGGGCGAGGUGAAUAAGAUCAAGAGUGCUCUGCUGAGCACUAA
CAAGGCUGUCGUGUCUCUGUCCAACGGCGUGUCUGUGCUGACCUUCAAGGUCCU
GGACCUGAAGAAUUACAUUGACAAGCAGCUCCUGCCCAUCCUGAACAAGCAGAG
CUGUUCUAUCUCCAAUAUUGAGACAGUUAUCGAGUUUCAGCAGAAGAACAACAG
GCUGCUUGAGAUCACACGGGAGUUCAGCGUGAACGCUGGGGUGACCACUCCCGU
GUCCACUUACAUGCUGACCAACUCAGAGCUGCUGUCCCUGAUCAACGACAUGCC
UAUUACAAACGACCAGAAGAAGUUGAUGAGCAACAACGUGCAGAUCGUGAGACA
GCAGUCUUACAGCAUCAUGUGCAUCAUCAAGGAGGAGGUGCUGGCGUACGUGGU
GCAGCUGCCCCUGUAUGGGGUCAUCGACACCCCUUGUUGGAAGCUGCACACUUC
UCCCCUGUGCACGACCAAUACGAAGGAGGGCAGCAACAUCUGUCUGACCCGGAC
AGAUCGGGGCUGGUAUUGCGACAAUGCCGGCUCCGUGUCCUUCUUCCCCCAGGC
UGAGACUUGCAAGGUGCAGAGUAACCGGGUGUUCUGCGACACCAUGAACUCCCG
GACUCUGCCCUCCGAGGUGAACCUGUGCAACGUGGACAUUUUCAACCCCAAGUA
CGAUUGCAAGAUCAUGACUAGCAAGACCGACGUGUCUUCUAGCGUGAUUACCAG
CCUGGGGGCGAUUGUGUCGUGCUACGGGAAGACGAAGUGUACAGCUUCCAACAA
GAACAGGGGCAUCAUCAAGACCUUCUCUAAUGGGUGCGACUACGUCAGCAAUAA
GGGCGUCGACACCGUCAGCGUGGGGAACACCUUGUACUGCGUGAACAAGCAGGA
GGGCAAGAGCCUGUACGUGAAGGGGGAGCCCAUUAUCAACUUCUACGACCCCCU
GGUGUUCCCCAGCGACGAGUUCGACGCCUCUAUCAGCCAGGUGAACGAGAAGAU
CAAUCAGUCCCUGGCCUUCAUUAGGAAGUCUGAUGAGCUGCUGCACAACGUGAA
CGCCGGGAAGAGCACUACGAAUAUCAUGAUCACGACGAUCAUCAUCGUGAUCAU
UGUGAUCCUGCUCAGCCUGAUCGCCGUGGGGCUGCUCCUGUACUGCAAGGCCAG
GAGCACCCCCGUGACCCUGUCCAAGGAUCAGCUGAGCGGGAUCAACAAUAUCGC
UUUCAGCAACUGAUAAGAAUUCUAGGCUGGAGCCUCGGUGGCCAUGCUUCUUGC
CCCUUGGGCCUCCCCCCAGCCCCUCCUCCCCUUCCUGCACCCGUACCCCCGUGGU
CUUUGAAUAAAGUCUGAGUGGGCGGCACUAGUAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAA
Sample 12 (encoding protein: eGFP, cap analog: m7GpppAmpU, other U in the sequence employs N1-methylpseudouridine (M1ψ) modification except for the grey marked UTP): SEQ ID NO: 12 
AUGGGCGGCGCAUGAGAGAAGCCCAGACUUGUUCUUUUUGCAGAAGCUCAGA
AUAAACGCUCAACUUUGGCGCCGCCACCAUGGUGAGCAAGGGCGAGGAGCUGUU
CACCGGGGUGGUGCCCAUCCUGGUCGAGCUGGACGGCGACGUAAACGGCCACAA
GUUCAGCGUGUCCGGCGAGGGCGAGGGCGAUGCCACCUACGGCAAGCUGACCCU
 
GAAGUUCAUCUGCACCACCGGCAAGCUGCCCGUGCCCUGGCCCACCCUCGUGACC
ACCCUGACCUACGGCGUGCAGUGCUUCAGCCGCUACCCCGACCACAUGAAGCAGC
ACGACUUCUUCAAGUCCGCCAUGCCCGAAGGCUACGUCCAGGAGCGCACCAUCU
UCUUCAAGGACGACGGCAACUACAAGACCCGCGCCGAGGUGAAGUUCGAGGGCG
ACACCCUGGUGAACCGCAUCGAGCUGAAGGGCAUCGACUUCAAGGAGGACGGCA
ACAUCCUGGGGCACAAGCUGGAGUACAACUACAACAGCCACAACGUCUAUAUCA
UGGCCGACAAGCAGAAGAACGGCAUCAAGGUGAACUUCAAGAUCCGCCACAACA
UCGAGGACGGCAGCGUGCAGCUCGCCGACCACUACCAGCAGAACACCCCCAUCGG
CGACGGCCCCGUGCUGCUGCCCGACAACCACUACCUGAGCACCCAGUCCGCCCUG
AGCAAAGACCCCAACGAGAAGCGCGAUCACAUGGUCCUGCUGGAGUUCGUGACC
GCCGCCGGGAUCACUCUCGGCAUGGACGAGCUGUACAAGUGAUAAGAAUUCUAG
GCUGGAGCCUCGGUGGCCAUGCUUCUUGCCCCUUGGGCCUCCCCCCAGCCCCUCC
UCCCCUUCCUGCACCCGUACCCCCGUGGUCUUUGAAUAAAGUCUGAGUGGGCGG
CACUAGUAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
Sample 13 (encoding protein: BA.4/5, cap analog: m7GpppAmpU, other U in the sequence employs N1-methylpseudouridine (M1ψ) modification except for the grey marked UTP): SEQ ID NO: 13 
AUGAAAUAAGGAUCCUUGUUCUUUUUGCAGAAGCUCAGAAUAAACGCUCAAC
UUUGGCGCCGCCACCAUGGACUGGACCUGGAUACUGUUCCUGGUGGCCGCCGCC
ACCAGAGUGCACAGCCGGGUCCAGCCAACCGAGAGCAUCGUGAGAUUCCCCAAC
AUAACAAACCUGUGCCCCUUCGACGAGGUGUUCAACGCCACCAGAUUCGCCAGC
GUGUACGCCUGGAACAGAAAAAGAAUCAGCAACUGCGUGGCCGACUACAGCGUG
CUGUACAACUUCGCCCCCUUCUUCGCCUUCAAAUGCUACGGCGUGAGCCCAACCA
AACUGAACGACCUGUGCUUCACCAACGUGUACGCCGACAGCUUCGUGAUCAGAG
GCAACGAGGUGAGCCAGAUCGCCCCAGGCCAGACCGGAAACAUCGCCGACUACA
ACUACAAACUGCCCGACGACUUCACCGGCUGCGUGAUCGCCUGGAACAGCAACA
AACUGGACAGCAAAGUGGGAGGCAACUACAACUACAGAUACAGACUGUUCAGAA
AAAGCAACCUGAAACCCUUCGAGCGAGACAUCAGCACCGAAAUCUACCAGGCCG
GCAACAAACCCUGCAACGGCGUGGCCGGGGUGAACUGCUACUUCCCCCUGCAGA
GCUACGGAUUCAGACCCACCUACGGCGUGGGGCACCAGCCCUACCGGGUGGUGG
UCCUGAGCUUCGAGCUGCUGCACGCCCCCGCAACCGUGUGCGGCCCCAAAAAAUC
GACCAACCUGGUGAAAAACAAAUGCGUGAACUUCUGAUAAGAAUUCUAGGCUGG
AGCCUCGGUGGCCAUGCUUCUUGCCCCUUGGGCCUCCCCCCAGCCCCUCCUCCCC
UUCCUGCACCCGUACCCCCGUGGUCUUUGAAUAAAGUCUGAGUGGGCGGCACUA
 
GUΛΛΛΛΛΛΛΛΛΛΛΛΛΛΛΛΛΛΛΛΛΛΛΛΛΛΛΛΛΛΛΛΛΛΛΛΛΛΛΛΛΛΛ

AAAAAAAAAAAAAAAAAAAAAAAAAAAAAAAAAAAAAAAAAAAAA

A
Sample 14 (encoding protein: RABV GP, cap analog: m7GpppAmpU, other U in the sequence employs N1-methylpseudouridine (M1ψ) modification except for the grey marked UTP): SEQ ID NO: 14 
AUGGGGAUCCAAGCUUAGCUUGCUUGUUCUUUUUGCAGAAGCUCAGAAUAA
ACGCUCAACUUUGGCGCCGCCACCAUGGUGCCUCAGGCCCUGUUGUUUGUGCCA
CUGCUGGGCUUUAGCCUGUGUUUUGGCAAAUUCCCAAUCUACACCAUCCCCGAU
GAGCUGGGCCCUUGGAGCCCUAUCGACAUCCACCAUCUCAGCUGCCCCAACAACC
UGGUGGUGGAAGAUGAGGGCUGCACCAAUCUGAGCGAGUUCAGCUACAUGGAAC
UGAAGGUGGGCUAUAUCAGCGCCAUCAAGGUGAACGGCUUCACCUGUACAGGCG
UCGUGACCGAGGCUGAGACAUACACCAACUUCGUGGGAUACGUGACAACCACCU
UCAAGAGAAAGCACUUCAGGCCAACACCUGACGCCUGCAGAGCCGCCUACAACU
GGAAGAUGGCCGGCGAUCCACGGUACGAGGAAAGCCUGCACAACCCUUAUCCUG
AUUACCACUGGCUGAGAACAGUGCGGACAACAAAGGAAAGCCUGAUUAUUAUCA
GCCCCAGCGUGACCGACCUGGACCCUUACGACAAGUCUCUGCACAGCAGAGUGU
UUCCUGGAGGGAAGUGCUCCGGCAUCACCGUGUCCUCUACCUACUGCAGCACCA
AUCACGACUACACCAUCUGGAUGCCUGAGAACCCCAGACCCAGAACCCCUUGCG
ACAUCUUCACCAACAGCCGCGGAAAACGCGCUUCUAAGGGCAACAAGACAUGCG
GCUUCGUCGACGAGAGAGGCCUGUACAAGAGCCUAAAAGGCGCUUGUAGACUGA
AGCUGUGCGGCGUGUUAGGCCUGAGACUGAUGGACGGCACGUGGGUCGCCAUGC
AGACCAGCGACGAGACAAAGUGGUGCCCUCCUGAUCAGCUGGUGAACCUGCAUG
AUUUCCGGAGCGACGAGAUCGAGCACCUGGUGGUGGAAGAACUUGUUAAGAAGC
GGGAAGAGUGCCUGGACGCCCUGGAAAGCAUCAUGACCACCAAGUCCGUGUCCU
UCAGACGGCUGAGCCACCUGCGGAAACUGGUUCCUGGCUUCGGCAAGGCCUAUA
CCAUCUUCAACAAGACACUGAUGGAAGCCGACGCUCACUACAAGAGCGUUAGAA
CCUGGAACGAGAUUAUCCCUUCUAAGGGCUGUCUGAAAGUGGGCGGCAGAUGCC
ACCCCCACGUGAAUGGCGUGUUCUUCAACGGCAUCAUCCUGGGACCUGAUGGCC
ACGUGCUGAUCCCCGAGAUGCAAAGCAGCCUGCUGCAGCAACACAUGGAACUGC
UGAAGUCCUCUGUGAUCCCACUGAUGCACCCUCUGGCCGAUCCUAGCACCGUGU
UCAAGGAAGGCGACGAGGCCGAGGACUUCGUGGAGGUGCACCUGCCUGACGUGU
ACAAGCAGAUCUCUGGCGUGGACCUGGGACUGCCCAAUUGGGGAAAAUACGUGC
UGAUGACCGCCGGAGCCAUGAUCGGCCUGGUGCUGAUCUUUAGCCUGAUGACAU
GGUGUCGGAGAGCCAACCGGCCUGAGUCCAAGCAGAGAAGCUUCGGCGGCACCG
 
GCAGAAACGUGUCUGUGACCAGCCAGUCUGGCAAGGUGAUCCCCAGCUGGGAGU
CUUACAAAUCUGGCGGACAGACAAGACUGUGAUAAGAAUUCUAGGCUGGAGCCU
CGGUGGCCAUGCUUCUUGCCCCUUGGGCCUCCCCCCAGCCCCUCCUCCCCUUCCU
GCACCCGUACCCCCGUGGUCUUUGAAUAAAGUCUGAGUGGGCGGCACUAGUAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAA
Sample 15 (encoding protein: RABV GP, cap analog: m7GpppAmpU, other U in the sequence employs N1-methylpseudouridine (M1ψ) modification except for the grey marked UTP): SEQ ID NO: 15 
AUGGGGAUCCAAGCUUAGCUUGCUUGUUCUUUUUGCAGAAGCUCAGAAUAA
ACGCUCAACUUUGGCGCCGCCACCAUGGUGCCGCAGGCCCUCCUGUUCGUGCCCC
UGCUGGGAUUUUCCCUUUGCUUCGGGAAGUUCCCAAUCUAUACGAUCCCCGACG
AGCUCGGGCCGUGGAGCCCCAUCGACAUCCACCACCUGAGUUGUCCCAACAACU
UGGUGGUGGAGGAUGAGGGCUGCACGAACCUCUCUGAGUUCAGCUACAUGGAGC
UGAAGGUGGGGUACAUCUCCGCCAUCAAGGUCAACGGCUUCACGUGCACGGGAG
UCGUGACCGAGGCGGAGACGUACACCAACUUCGUUGGGUACGUGACCACCACCU
UCAAGAGGAAGCAUUUUCGGCCCACGCCCGACGCUUGUCGGGCGGCGUAUAAUU
GGAAGAUGGCCGGGGACCCUCGGUACGAGGAGUCCCUGCACAAUCCUUACCCUG
ACUACCACUGGCUGCGGACUGUGAGGACCACGAAGGAGUCACUGAUCAUCAUUU
CGCCCAGUGUCACCGAUCUGGACCCGUACGACAAGUCACUGCACUCCAGGGUGU
UCCCUGGAGGCAAGUGCUCCGGGAUCACAGUGAGCAGCACGUACUGCAGCACCA
ACCACGACUAUACCAUCUGGAUGCCAGAGAAUCCUCGGCCUCGUACCCCCUGCG
ACAUCUUUACAAACAGUCGGGGGAAGCGGGCGAGCAAGGGGAACAAGACCUGCG
GCUUCGUGGAUGAGCGCGGCCUGUACAAGAGCCUGAAGGGGGCUUGCCGUCUGA
AGCUGUGUGGCGUGCUCGGGCUCCGUCUGAUGGACGGGACCUGGGUCGCCAUGC
AGACUUCAGACGAGACAAAGUGGUGCCCUCCCGACCAGCUGGUCAAUCUCCACG
ACUUCAGGUCCGAUGAGAUCGAGCACCUGGUCGUGGAGGAGCUCGUCAAGAAGC
GCGAGGAGUGCCUCGACGCUCUUGAGAGCAUCAUGACCACCAAGAGCGUCAGCU
UCCGCAGGCUGAGCCACCUGCGGAAGCUGGUGCCUGGGUUCGGGAAGGCCUACA
CCAUCUUCAACAAGACCCUGAUGGAGGCCGACGCGCACUACAAGAGCGUGCGGA
CCUGGAAUGAGAUCAUUCCAUCCAAGGGCUGUCUGAAGGUGGGUGGGCGGUGCC
ACCCACACGUCAACGGCGUGUUCUUCAACGGGAUCAUCCUCGGGCCUGACGGGC
ACGUGCUCAUCCCGGAGAUGCAGAGCUCGCUGCUCCAGCAGCACAUGGAGCUGC
UGAAGAGCAGCGUGAUUCCCCUGAUGCAUCCGCUCGCUGAUCCCUCGACUGUGU
 
UUAAGGAGGGGACGAGGCCGAGGAUUUCGUGGAGGUCCACCUCCCUGACGUCU
ACAAGCAGAUCAGCGGGGUGGACCUUGGGCUCCCAAACUGGGGCAAGUACGUGC
UGAUGACAGCCGGGGCCAUGAUCGGCCUCGUGCUGAUCUUCUCACUGAUGACCU
GGUGUCGGCGGGCCAAUCGGCCCGAGAGCAAGCAGCGCUCUUUCGGGGGCACUG
GGCGGAAUGUGUCAGUGACUUCUCAGUCCGGGAAGGUGAUACCUUCCUGGGAGU
CCUACAAGUCCGGCGGCCAGACGCGGCUGUGAUAAGAAUUCUAGGCUGGAGCCU
CGGUGGCCAUGCUUCUUGCCCCUUGGGCCUCCCCCCAGCCCCUCCUCCCCUUCCU
GCACCCGUACCCCCGUGGUCUUUGAAUAAAGUCUGAGUGGGCGGCACUAGUAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAA
Sample 16 (encoding protein: RABV GP, cap analog: m7GpppAmpU, other U in the sequence employs N1-methylpseudouridine (M1ψ) modification except for the grey marked UTP): SEQ ID NO: 16 
AUGGGGAUCCCUGAACUGGGUUAGGUGCCGCUGUUGCUGCUCGUGUUGAAUC
UAGAACCGUAGCCAGACGCCACCAUGGUGCCGCAGGCCCUCCUGUUCGUGCCCCU
GCUGGGAUUUUCCCUUUGCUUCGGGAAGUUCCCAAUCUAUACGAUCCCCGACGA
GCUCGGGCCGUGGAGCCCCAUCGACAUCCACCACCUGAGUUGUCCCAACAACUU
GGUGGUGGAGGAUGAGGGCUGCACGAACCUCUCUGAGUUCAGCUACAUGGAGCU
GAAGGUGGGGUACAUCUCCGCCAUCAAGGUCAACGGCUUCACGUGCACGGGAGU
CGUGACCGAGGCGGAGACGUACACCAACUUCGUUGGGUACGUGACCACCACCUU
CAAGAGGAAGCAUUUUCGGCCCACGCCCGACGCUUGUCGGGCGGCGUAUAAUUG
GAAGAUGGCCGGGGACCCUCGGUACGAGGAGUCCCUGCACAAUCCUUACCCUGA
CUACCACUGGCUGCGGACUGUGAGGACCACGAAGGAGUCACUGAUCAUCAUUUC
GCCCAGUGUCACCGAUCUGGACCCGUACGACAAGUCACUGCACUCCAGGGUGUU
CCCUGGAGGCAAGUGCUCCGGGAUCACAGUGAGCAGCACGUACUGCAGCACCAA
CCACGACUAUACCAUCUGGAUGCCAGAGAAUCCUCGGCCUCGUACCCCCUGCGAC
AUCUUUACAAACAGUCGGGGGAAGCGGGCGAGCAAGGGGAACAAGACCUGCGGC
UUCGUGGAUGAGCGCGGCCUGUACAAGAGCCUGAAGGGGGCUUGCCGUCUGAAG
CUGUGUGGCGUGCUCGGGCUCCGUCUGAUGGACGGGACCUGGGUCGCCAUGCAG
ACUUCAGACGAGACAAAGUGGUGCCCUCCCGACCAGCUGGUCAAUCUCCACGAC
UUCAGGUCCGAUGAGAUCGAGCACCUGGUCGUGGAGGAGCUCGUCAAGAAGCGC
GAGGAGUGCCUCGACGCUCUUGAGAGCAUCAUGACCACCAAGAGCGUCAGCUUC
CGCAGGCUGAGCCACCUGCGGAAGCUGGUGCCUGGGUUCGGGAAGGCCUACACC
AUCUUCAACAAGACCCUGAUGGAGGCCGACGCGCACUACAAGAGCGUGCGGACC
 
UGGAAUGAGAUCAUUCCAUCCAAGGGCUGUCUGAAGGUGGGUGGCGGUGCCAC
CCACACGUCAACGGCGUGUUCUUCAACGGGAUCAUCCUCGGGCCUGACGGCAC
GUGCUCAUCCCGGAGAUGCAGAGCUCGCUGCUCCAGCAGCACAUGGAGCUGCUG
AAGAGCAGCGUGAUUCCCCUGAUGCAUCCGCUCGCUGAUCCCUCGACUGUGUUU
AAGGAGGGGGACGAGGCCGAGGAUUUCGUGGAGGUCCACCUCCCUGACGUCUAC
AAGCAGAUCAGCGGGGUGGACCUUGGGCUCCCAAACUGGGGCAAGUACGUGCUG
AUGACAGCCGGGGCCAUGAUCGGCCUCGUGCUGAUCUUCUCACUGAUGACCUGG
UGUCGGCGGGCCAAUCGGCCCGAGAGCAAGCAGCGCUCUUUCGGGGGCACUGGG
CGGAAUGUGUCAGUGACUUCUCAGUCCGGGAAGGUGAUACCUUCCUGGGAGUCC
UACAAGUCCGGCGGCCAGACGCGGCUGUGAUAAGAAUUCUAGGCUGGAGCCUCG
GUGGCCAUGCUUCUUGCCCCUUGGGCCUCCCCCCAGCCCCUCCUCCCCUUCCUGC
ACCCGUACCCCCGUGGUCUUUGAAUAAAGUCUGAGUGGGCGGCACUAGUAAAA
AAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAA
AAAAA
Sample 17 (encoding protein: RABV GP, cap analog: m7GpppAmpU, other U in the sequence employs N1-methylpseudouridine (M1ψ) modification except for the grey marked UTP): SEQ ID NO: 17 
AUGGGGAUCCCUGAACUGGGUUAGGUGCCGCUGUUGCUGCUCGUGUUGAAUC
UAGAACCGUAGCCAGACGCCACCAUGGUGCCCCAGGCCCUGCUGUUCGUGCCUC
UGCUGGGAUUCAGCCUGUGCUUCGGGAAGUUCCCAAUUUACACCAUCCCCGAUG
AGCUGGGCCCUUGGUCCCCCAUCGACAUCCACCACCUGAGUUGCCCCAACAAUCU
GGUGGUGGAGGAUGAGGGGUGCACCAACCUGAGCGAGUUCAGCUACAUGGAGCU
GAAGGUGGGGUACAUCUCUGCCAUCAAGGUGAACGGCUUCACCUGCACCGGCGU
GGUGACUGAGGCAGAGACAUACACCAACUUCGUGGGCUAUGUGACCACCACCUU
CAAGCGGAAGCACUUCAGGCCCACCCCCGACGCCUGCCGCGCCGCCUAUAACUGG
AAGAUGGCCGGGGACCCUCGGUACGAGGAGUCCCUGCACAAUCCUUACCCAGAU
UAUCACUGGCUGCGGACAGUGCGGACCACCAAGGAGUCUCUGAUCAUCAUCAGC
CCUAGCGUGACAGACCUGGACCCUUACGAUAAGAGCCUGCACAGCAGGGUGUUC
CCCGGCGGCAAGUGCAGCGGCAUCACCGUGUCCAGCACCUACUGCAGCACCAACC
ACGACUACACCAUCUGGAUGCCAGAGAACCCCAGGCCCCGCACCCCCUGCGACAU
CUUCACAAAUUCCAGGGGGAAGCGGGCCUCCAAGGGCAAUAAGACUUGCGGGUU
CGUGGAUGAGCGGGGCCUGUACAAGUCCCUGAAGGGGGCUUGCAGGCUGAAGCU
GUGCGGGGUGCUGGGCCUGAGGCUGAUGGACGGCACCUGGGUCGCCAUGCAGAC
CUCAGACGAGACAAAGUGGUGCCCUCCUGACCAGCUGGUGAACCUGCACGACUU
 
CAGGAGCGAUGAGAUCGAGCACCUGGUGGUGGAGGAGCUCGUGAAGAAGAGGG
AGGAGUGCCUGGACGCUCUGGAGUCCAUCAUGACUACCAAGAGCGUCAGCUUCC
GCAGGCUGAGCCACCUGCGGAAGCUGGUGCCAGGCUUCGGCAAGGCCUACACCA
UCUUCAACAAGACCCUGAUGGAGGCCGACGCUCACUACAAGAGCGUGCGGACCU
GGAAUGAGAUCAUUCCAUCCAAGGGCUGUCUGAAGGUGGGAGGCCGCUGCCACC
CCCACGUCAACGGCGUGUUCUUCAACGGGAUCAUCCUGGGGCCUGACGGGCACG
UGCUGAUCCCAGAGAUGCAGAGCAGCCUGCUGCAGCAGCACAUGGAGCUGCUGA
AGUCCAGCGUGAUCCCACUGAUGCAUCCCCUGGCCGACCCCUCAACUGUGUUCA
AGGAGGGGGACGAGGCCGAGGACUUCGUGGAGGUCCACCUGCCUGACGUGUACA
AGCAGAUCAGCGGCGUGGACCUGGGGCUGCCCAACUGGGGCAAGUACGUGCUGA
UGACCGCCGGCGCCAUGAUCGGUCUGGUGCUGAUCUUCAGCCUGAUGACAUGGU
GCCGGCGGGCCAACCGGCCCGAGAGCAAGCAGCGCUCUUUCGGGGGCACCGGCC
GGAACGUGUCCGUGACCUCCCAGUCUGGGAAGGUGAUCCCCAGCUGGGAGAGCU
ACAAGUCCGGCGGCCAGACCCGGCUGUGAUAAGAAUUCUAGGCUGGAGCCUCGG
UGGCCAUGCUUCUUGCCCCUUGGGCCUCCCCCCAGCCCCUCCUCCCCUUCCUGCA
CCCGUACCCCCGUGGUCUUUGAAUAAAGUCUGAGUGGGCGGCACUAGUAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAA
Sample 18 (encoding protein: Luc, cap analog: m7GpppAmpG): SEQ ID NO: 18 
AGGGGGAUCCAAGCUUAGCUUGCUUGUUCUUUUUGCAGAAGCUCAGAAUAA
ACGCUCAACUUUGGCGCCGCCACCAUGGAGGACGCCAAAAACAUCAAAAAAGGC
CCCGCCCCCUUCUACCCCCUGGAGGACGGCACCGCCGGAGAGCAGCUGCACAAAG
CCAUGAAAAGAUACGCCCUGGUCCCCGGCACCAUCGCCUUCACCGACGCCCACAU
CGAGGUGGACAUCACCUACGCCGAGUACUUCGAGAUGAGCGUGAGACUGGCCGA
AGCCAUGAAAAGGUACGGCCUGAACACAAACCACAGAAUCGUGGUGUGCAGCGA
GAACAGCCUGCAGUUCUUCAUGCCCGUGCUGGGAGCCCUGUUCAUCGGCGUGGC
CGUGGCCCCCGCCAACGACAUCUACAACGAGAGAGAGCUGCUCAACAGCAUGGG
CAUCAGCCAGCCCACCGUGGUGUUCGUGAGCAAAAAAGGCCUGCAGAAAAUCCU
GAACGUGCAGAAAAAACUCCCCAUCAUCCAGAAAAUCAUCAUCAUGGAUAGCAA
AACCGACUACCAGGGCUUCCAGAGCAUGUACACCUUCGUGACCAGCCACCUGCCC
CCUGGCUUCAACGAGUACGACUUCGUGCCGGAGAGCUUCGACAGGGACAAAACC
AUCGCCCUGAUCAUGAACAGCAGCGGCAGCACCGGCCUGCCCAAAGGCGUGGCC
CUGCCCCACCGGACAGCCUGCGUGAGAUUCAGCCACGCCAGAGAUCCCAUCUUCG
GCAACCAGAUCAUCCCCGACACCGCCAUCCUGAGCGUGGUCCCAUUCCACCACGG
 
CUUCGGCAUGUUCACCACCCUGGGCUACCUGAUCUGCGGCUUCCGCGUGGUGCU
GAUGUACAGAUUCGAGGAGGAGCUGUUCCUAAGAAGUCUGCAGGACUACAAAAU
CCAGAGCGCCCUGCUGGUGCCCACCCUGUUCAGCUUCUUCGCCAAAAGCACCCUG
AUCGACAAAUACGACCUGAGCAACCUGCACGAGAUCGCCAGCGGCGGCGCCCCA
CUGAGCAAAGAGGUGGGCGAGGCCGUGGCCAAAAGAUUCCACCUGCCCGGAAUC
AGACAGGGCUACGGCCUGACGGAAACCACCAGCGCCAUCCUGAUCACCCCCGAG
GGCGACGACAAACCCGGCGCCGUGGGCAAAGUGGUGCCCUUCUUCGAGGCCAAA
GUGGUGGACCUGGACACCGGCAAAACCCUGGGAGUGAACCAGAGAGGGGAGCUG
UGCGUGAGAGGCCCCAUGAUCAUGAGCGGCUACGUGAACAACCCCGAGGCCACC
AACGCCCUCAUCGACAAAGACGGCUGGCUGCACAGCGGCGACAUCGCCUACUGG
GACGAGGACGAGCACUUCUUCAUCGUGGACCGCCUGAAAUCGCUGAUCAAAUAC
AAAGGAUACCAGGUGGCCCCCGCCGAGCUGGAGAGCAUCCUGCUGCAGCACCCC
AACAUCUUCGACGCCGGCGUGGCCGGCCUGCCCGACGACGACGCCGGCGAGCUGC
CCGCCGCCGUCGUGGUGCUGGAGCACGGAAAAACCAUGACCGAGAAAGAGAUCG
UGGACUACGUGGCCAGCCAGGUGACCACAGCCAAAAAACUGAGGGGCGGCGUGG
UGUUCGUGGACGAGGUGCCCAAAGGCCUGACCGGCAAACUGGACGCCCGGAAAA
UCAGAGAGAUCCUGAUCAAAGCCAAAAAAGGCGGCAAAAUCGCCGUGUGAUAAG
AAUUCUAGGCUGGAGCCUCGGUGGCCAUGCUUCUUGCCCCUUGGGCCUCCCCCC
AGCCCCUCCUCCCCUUCCUGCACCCGUACCCCCGUGGUCUUUGAAUAAAGUCUGA
GUGGGCGGCACUAGUAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AA
Sample 19 (encoding protein: BA.4/5, cap analog: m7GpppAmpG): SEQ ID NO: 19 
AGGAUCCUUGUUCUUUUUGCAGAAGCUCAGAAUAAACGCUCAACUUUGGCGC
CGCCACCAUGGACUGGACCUGGAUACUGUUCCUGGUGGCCGCCGCCACCAGAGU
GCACAGCCGGGUCCAGCCAACCGAGAGCAUCGUGAGAUUCCCCAACAUAACAAA
CCUGUGCCCCUUCGACGAGGUGUUCAACGCCACCAGAUUCGCCAGCGUGUACGC
CUGGAACAGAAAAAGAAUCAGCAACUGCGUGGCCGACUACAGCGUGCUGUACAA
CUUCGCCCCCUUCUUCGCCUUCAAAUGCUACGGCGUGAGCCCAACCAAACUGAAC
GACCUGUGCUUCACCAACGUGUACGCCGACAGCUUCGUGAUCAGAGGCAACGAG
GUGAGCCAGAUCGCCCCAGGCCAGACCGGAAACAUCGCCGACUACAACUACAAA
CUGCCCGACGACUUCACCGGCUGCGUGAUCGCCUGGAACAGCAACAAACUGGAC
AGCAAAGUGGGAGGCAACUACAACUACAGAUACAGACUGUUCAGAAAAAGCAAC
 
CUGAAACCCUUCGAGCGAGACAUCAGCACCGAAAUCUACCAGGCCGGCAACAAA
CCCUGCAACGGCGUGGCCGGGGUGAACUGCUACUUCCCCCUGCAGAGCUACGGA
UUCAGACCCACCUACGGCGUGGGCACCAGCCCUACCGGGUGGUGGUCCUGAGC
UUCGAGCUGCUGCACGCCCCCGCAACCGUGUGCGGCCCAAAAAAUCGACCAACC
UGGUGAAAAACAAAUGCGUGAACUUCUGAUAAGAAUUCUAGGCUGGAGCCUCGG
UGGCCAUGCUUCUUGCCCCUUGGGCCUCCCCCCAGCCCCUCCUCCCCUUCCUGCA
CCCGUACCCCCGUGGUCUUUGAAUAAAGUCUGAGUGGGCGGCACUAGUAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
Sample 20 (encoding protein: Epo, cap analog: m7GpppAmpG): SEQ ID NO: 20

AGGGGGAUCCAAGCUUAGCUUGCUUGUUCUUUUUGCAGAAGCUCAGAAUAA
ACGCUCAACUUUGGCGCCGCCACCAUGGGUGUGCAUGAGUGUCCCGCUUGGCUU
UGGCUGUUAUUAUCAUUACUUUCCUUACCUCUUGGCCUGCCUGUCUUAGGCGCC
CCUCCUCGUUUAAUCUGUGAUAGCCGUGUGUUAGAGAGAUACUUGCUUGAGGCU
AAAGAGGCCGAGAAUAUUACUACCGGUUGUGCUGAACAUUGCUCGCUGAACGAG
AACAUUACCGUGCCCGACACCAAAGUUAACUUCUAUGCUUGGAAACGUAUGGAG
GUGGGUCAGCAGGCCGUUGAGGUAUGGCAGGGUUUGGCCCUUCUUUCGGAGGCC
GUGUUACGUGGUCAGGCUCUCUUGGUUAACUCUUCUCAGCCUUGGGAGCCUUUA
CAGUUACAUGUUGAUAAAGCCGUUAGCGGCUUGAGAAGUUUGACCACUUUGCUU
CGGGCUCUGGGUGCUCAAAAAGAGGCUAUUUCACCCCCUGAUGCUGCUAGCGCU
GCUCCACUUAGAACCAUCACUGCCGACACUUUUCGUAAAUUGUUUCGUGUUUAU
AGCAAUUUCUUACGUGGCAAACUGAAAUUAUACACUGGCGAGGCUUGCAGAACU
GGUGACAGAUGAUAAGAAUUCUAGGCUGGAGCCUCGGUGGCCAUGCUUCUUGCC
CCUUGGGCCUCCCCCCAGCCCCUCCUCCCCUUCCUGCACCCGUACCCCCGUGGUC
UUUGAAUAAAGUCUGAGUGGGCGGCACUAGUAAAAAAAAAAAAAAAAAAAAA
AAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAA

## Claims

1. A method for separating mRNA molecules, comprising one or more magnetic particle separation steps and one or more positive-pressure affinity chromatography separation steps.

2. The method according to claim 1, wherein the method comprises first performing one or more magnetic particle separation steps, then performing one or more positive-pressure affinity chromatography separation steps, followed by optionally one or more magnetic particle separation steps; or wherein the method comprises first performing one or more positive-pressure affinity chromatography separation steps followed by one or more magnetic particle separation steps.

3. A method for separating mRNA molecules, comprising the steps of:
(a) providing magnetic particles comprising ligands capable of binding to the mRNA molecules;
(b) contacting a solution containing the mRNA molecules with the magnetic particles to obtain the magnetic particles comprising the bound mRNA molecules;
(c) separating the magnetic particles comprising the bound mRNA molecules using a magnetic field;
(d) eluting the mRNA molecules from the separated magnetic particles to obtain a primary purified solution containing the mRNA molecules; and
(e) loading the solution containing the mRNA molecules obtained in step (d) onto an affinity chromatography column comprising oligo (dT), and eluting the mRNA molecules in a positive-pressure manner to obtain a secondary purified solution containing the mRNA molecules.

4. The method according to claim 3, further comprising:
a step of performing in vitro transcription on a DNA template prior to step (b) to generate a solution containing the mRNA molecules; and/or
a step of capping and/or tailing the mRNA molecules prior to step (b); and/or
a step of capping and/or tailing the mRNA molecules prior to step (e); and/or
a step of capping the mRNA molecules after step (e); and/or
a step of performing buffer exchange and concentration, and/or sterilization and filtration on the secondary purified solution containing the mRNA molecules after step (e), optionally wherein the buffer exchange and concentration step is performed by using a LiCl method and/or a magnetic particle method, optionally wherein the magnetic particle method comprises repeating the steps (a)-(d).

5. A method for separating mRNA molecules, comprising the steps of:
(a) providing a solution containing the mRNA molecules;
(b) loading the solution containing the mRNA molecules onto an affinity chromatography column comprising oligo (dT), eluting the mRNA molecules in a positive-pressure manner to obtain a primary purified solution containing the mRNA molecules;
(c) providing magnetic particles comprising ligands capable of binding to the mRNA molecules, and contacting the solution containing the mRNA molecules obtained in step (b) with the magnetic particles to obtain the magnetic particles comprising the bound mRNA molecules;
(d) separating the magnetic particles comprising the bound mRNA molecules using a magnetic field; and
(e) eluting the mRNA molecules from the separated magnetic particles to obtain a secondary purified solution containing the mRNA molecules.

6. The method according to claim 5, wherein
step (a) further comprises performing in vitro transcription on a DNA template to generate a solution containing the mRNA molecules; and/or
step (a) further comprises capping and/or tailing the mRNA molecules; and/or
the method further comprises a step of capping the mRNA molecules after step (b); and/or
the method further comprises a step of capping the mRNA molecules after step (e); and/or
the method further comprises a step of performing buffer exchange and concentration, and/or sterilization and filtration on the secondary purified solution containing the mRNA molecules after step (e), optionally wherein the buffer exchange and concentration step is performed by using a LiCl method and/or a magnetic particle method, optionally wherein the magnetic particle method comprises repeating the steps (c)-(e).

7. The method according to any one of claims 1 to 6,
wherein the magnetic particles are carboxyl magnetic beads, preferably Dynabeads^{™} MyOne^{™} carboxylic acid magnetic beads; and/or
wherein the positive-pressure affinity chromatography comprises Oligo(dT)25 affinity packing, preferably POROS^{™} Oligo(dT)25 affinity packing; and/or
wherein the method is performed in an automated, semi-automated or manual manner; and/or
wherein the mRNA molecules comprise one or more functional nucleotide analogs, optionally wherein the functional nucleotide analogs are one or more selected from: pseudouridine, 1-methyl-pseudouridine, and 5-methylcytosine.

8. A system for separating mRNA molecules, comprising:
a first purification module comprising magnetic particles, wherein the magnetic particles comprise ligands capable of binding to the mRNA molecules;
a second purification module comprising an affinity chromatography column, wherein the affinity chromatography column is configured to operate in a positive-pressure mode; and
a fluid module comprising a container for containing a solution containing the mRNA, wherein the fluid module is in fluid communication with the first purification module and the second purification module.

9. The system according to claim 8, wherein the system further comprises a feed module configured to provide a source of the mRNA molecules, and/or a collection module configured to collect the mRNA molecules.

10. The system according to claim 8, wherein the fluid module further comprises a liquid transfer device, optionally wherein the liquid transfer device is a pipettor, a pipette or a mobile workstation; and/or
wherein the fluid module further comprises a conduit that facilitates fluid communication of the first purification module, the second purification module, and the fluid module; and/or
wherein the fluid module further comprises a container for containing an elution solution for eluting the mRNA from the magnetic particles, and a container for containing an elution solution for eluting the mRNA from the affinity chromatography column; and/or
wherein the fluid module further comprises a container for containing a wash solution for washing the magnetic particles, and a container for containing a wash solution for washing the affinity chromatography column.

11. The system according to claim 8, wherein the first purification module further comprises a magnetic separator or a magnetic field.

12. The system according to claim 8, wherein the system further comprises one or more additional modules including, but not limited to, one or more devices configured to perform the following operations:
performing in vitro transcription on DNA to generate a solution containing the mRNA molecules;
capping and/or tailing the mRNA molecules;
performing buffer exchange and concentration on a solution containing the mRNA molecules; and/or
performing sterilization and filtration on a solution containing the mRNA molecules.

13. The system according to claim 12,
wherein the buffer exchange and concentration is performed by using a LiCl method; or
wherein the buffer exchange and concentration is performed by using a magnetic particle method, wherein a module for performing buffer exchange and concentration is the same as or different from the first purification module.

14. The system according to any one of claims 8 to 13, wherein the system is configured to operate in an automated, semi-automated or manual manner, optionally wherein the system further comprises a control module configured to control various modules of the system to operate in an automated or semi-automated manner.

15. The system according to any one of claims 8-14, wherein the mRNA molecules comprise one or more functional nucleotide analogs, optionally wherein the functional nucleotide analogs are one or more selected from: pseudouridine, 1-methyl-pseudouridine, and 5-methylcytosine.
